(19) Europäisches Patentamt
European Patent Office
Office européen des brevets

(11) **EP 2 327 799 A1**

(12) **EUROPEAN PATENT APPLICATION**
published in accordance with Art. 153(4) EPC

(43) Date of publication:
**01.06.2011 Bulletin 2011/22**

(51) Int Cl.:
*C12Q 1/68* (2006.01)   *G01N 33/58* (2006.01)
*C12N 15/09* (2006.01)   *G01N 33/53* (2006.01)

(21) Application number: **09808329.8**

(22) Date of filing: **18.08.2009**

(86) International application number:
**PCT/JP2009/064686**

(87) International publication number:
**WO 2010/021394 (25.02.2010 Gazette 2010/08)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **19.08.2008 JP 2008210467**

(71) Applicant: **Sumitomo Chemical Company, Limited Tokyo 104-8260 (JP)**

(72) Inventors:
• **TOMIGAHARA, Yoshitaka**
  **Toyonaka-shi**
  **Osaka 560-0013 (JP)**
• **SATOH, Hideo**
  **Ibaraki-shi**
  **Osaka 567-0826 (JP)**
• **TARUI, Hirokazu**
  **Toyonaka-shi**
  **Osaka 561-0802 (JP)**

(74) Representative: **Ford, Hazel**
  **J.A. Kemp & Co.**
  **14 South Square**
  **Gray's Inn**
  **London WC1R 5JJ (GB)**

(54) **METHOD FOR QUANTIFICATION OR DETECTION OF DNA**

(57)   The present invention relates to a method for quantifying or detecting DNA having a target DNA region, and so on.

EP 2 327 799 A1

**Description**

TECHNICAL FIELD

The present invention relates to a method for quantifying or detecting DNA comprising a target DNA region contained in a specimen, and so on.

BACKGROUND ART

Known as a method for detecting DNA comprising a target DNA region contained in a specimen are, for example, a method of detecting DNA amplified by a chain reaction of DNA synthesis by DNA polymerase (Polymerase Chain Reaction; hereinafter, sometimes referred to as PCR) after extraction of biological genomic DNA contained in a specimen, a method of detecting DNA by hybridization of a fluorescent-labeled oligonucleotide with a target DNA region possessed by DNA in a biological specimen, and so on (see, for example, J. Cataract. Refract. Surg., 2007;33(4):635-641, and Environ. Mol. Mutagen., 1991;18(4):259-262).

DISCLOSURE OF THE INVENTION

It is an object of the present invention to provide a method for quantifying or detecting DNA comprising a target DNA region contained in a specimen in a simple and convenient manner.
The present invention provides:

[Invention 1]

A method for quantifying or detecting DNA which comprises a target DNA region and is contained in a specimen, comprising:

(1) A first step of preparing a specimen containing a test oligonucleotide that is DNA comprising a target DNA region;
(2) A second step of mixing the test oligonucleotide contained in the specimen prepared in the first step and a detection oligonucleotide that is capable of complementarily binding with the test oligonucleotide and has a plurality of identification functions, to form a detection complex comprising the test oligonucleotide and the detection oligonucleotide, and immobilizing the detection complex to a support; and
(3) A third step of quantifying or detecting said DNA comprising a target DNA region by detecting the detection oligonucleotide contained in the detection complex formed in the second step by its identification function (hereinafter, sometimes referred to as the present method);

[Invention 2]

The method according to invention 1, wherein the detection oligonucleotide having a plurality of identification functions is a composite detection oligonucleotide comprising a plurality of complementarily bound oligonucleotides, or a composite detection oligonucleotide comprising a methylated oligonucleotide having a plurality of methylation sites;

[Invention 3]

The method according to invention 2, wherein the composite detection oligonucleotide comprises an oligonucleotide comprising methylated DNA;

[Invention 4]

The method according to invention 2 or 3, wherein the identification function of the composite detection oligonucleotide is identification function of a bound methylated DNA antibody;

[Invention 5]

The method according to invention 3 or 4, wherein the methylated DNA is 5-methylcytosine;

[Invention 6]

The method according to invention 4 or 5, wherein the methylated DNA antibody is methylcytosine antibody;

[Invention 7]

The method according to any one of inventions 1 to 6, wherein the specimen is any of the following biological specimens:

(a) mammalian blood, a body fluid, excreta, a body secretion, a cell lysate or a tissue lysate,
(b) DNA extracted from one selected from the group consisting of mammalian blood, a body fluid, excreta, a body secretion, a cell lysate or a tissue lysate,
(c) DNA prepared using RNA extracted from one selected from the group consisting of mammalian tissue, cell, tissue lysate or cell lysate as a template,
(d) DNA extracted from a bacterium, a fungus or a virus, or
(f) DNA prepared using RNA extracted from a bacterium, a fungus, or a virus as a template;

[Invention 8]

A method for labeling a specimen using the composite detection oligonucleotide used in the method of any one of inventions 2 to 7;

[Invention 9]

A method for labeling a specimen using the composite detection oligonucleotide used in the method of any one of inventions 2 to 7 and a reagent capable of labeling the composite detection oligonucleotide;

[Invention 10]

The method according to invention 8 or 9, wherein an object to be labeled is DNA or protein;

[Invention 11]

The method according to any one of inventions 1 to 10, wherein in the second step, the detection complex and the support are bound via a specific oligonucleotide that does not inhibit binding of the test oligonucleotide and the detection oligonucleotide, and is capable of complementarily binding with the test oligonucleotide;

[Invention 12]

The method according to any one of inventions 1 to 11, wherein the DNA comprising a target DNA region is any of the following DNA comprising a target DNA region:

(a) DNA digested in advance with a restriction enzyme recognition cleavage site for which is not present in the target DNA region,
(b) DNA comprising a target DNA region and being purified in advance,
(c) free DNA comprising a target DNA region in blood,
(d) DNA comprising a target DNA region and being derived from microbial genome, or
(e) DNA comprising a target DNA region and having been generated from RNA by a reverse transcriptase;

[Invention 13]

A method for quantifying or detecting DNA which comprises a target DNA region and is contained in a specimen, comprising:

(1) A first step of preparing a specimen containing a test oligonucleotide that is DNA comprising a target DNA region,
(2) A second step of mixing the test oligonucleotide contained in the specimen prepared in the first step, and a detection oligonucleotide that is capable of complementarily binding with the test oligonucleotide and comprises a methylated oligonucleotide, to form a detection complex comprising the test oligonucleotide and the detection oligonucleotide, and immobilizing the detection complex to a support, and

(3) A third step of quantifying or detecting said DNA comprising a target DNA region by detecting the detection oligonucleotide contained in the detection complex formed in the second step by its identification function; and

[Invention 14]

The method according to invention 13, wherein the identification function employs a methylated DNA antibody or methylcytosine antibody;
and so on.

Brief description of drawings

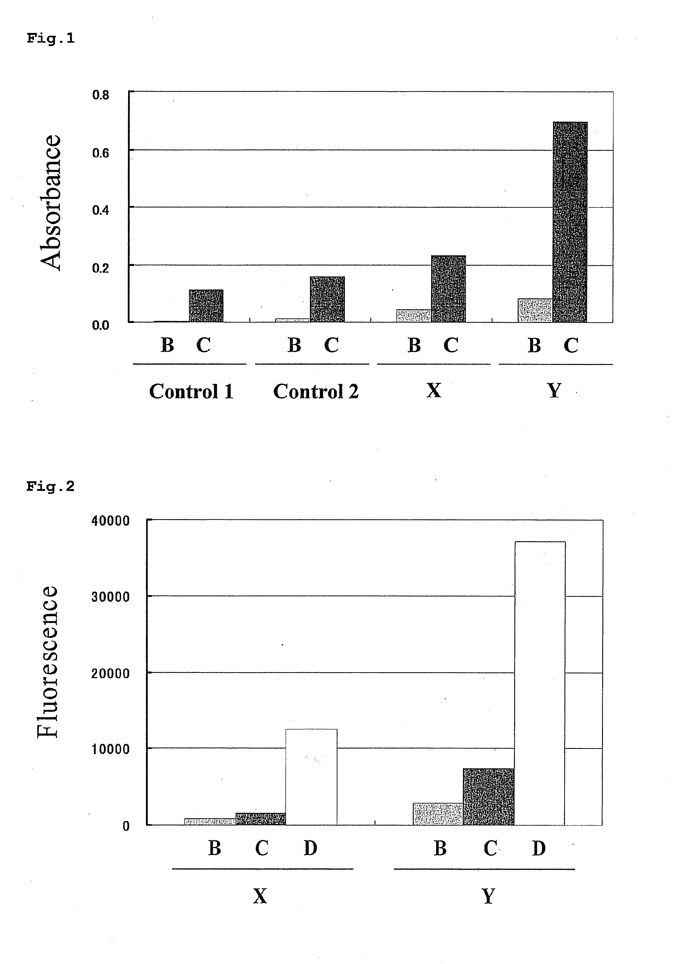

Fig. 1 is a figure showing a result of an experiment for detecting a test oligonucleotide by conducting Control 1 treatment (using 5'-end FITC-labeled oligonucleotide), Control 2 treatment (using 3'-end FLC-labeled oligonucleotide), X treatment (using methylated oligonucleotide M1) and Y treatment (using methylated oligonucleotide M12) in Example 1. In the figure, B represents a value of absorbance measured for a test oligonucleotide concentration of 0.001 pmol/10 μL TE buffer solution. C represents a value of absorbance measured for a test oligonucleotide concentration of 0.01 pmol DNA/10 μL TE buffer solution.

Fig. 2 is a figure showing a result of an experiment for detecting a test oligonucleotide by conducting X treatment (using methylated oligonucleotide M1) and Y treatment (using methylated oligonucleotide M12) in Example 2. In the figure,B represents a value of fluorescence measured for a test oligonucleotide concentration of 0.0001 pmol/ 10 L TE buffer solution. C represents a value of fluorescence measured for a test oligonucleotide concentration of 0.001 pmol/10 μL TE buffer solution. D represents a value of fluorescence measured for a test oligonucleotide concentration of 0.01 pmol/10 μL TE buffer solution.

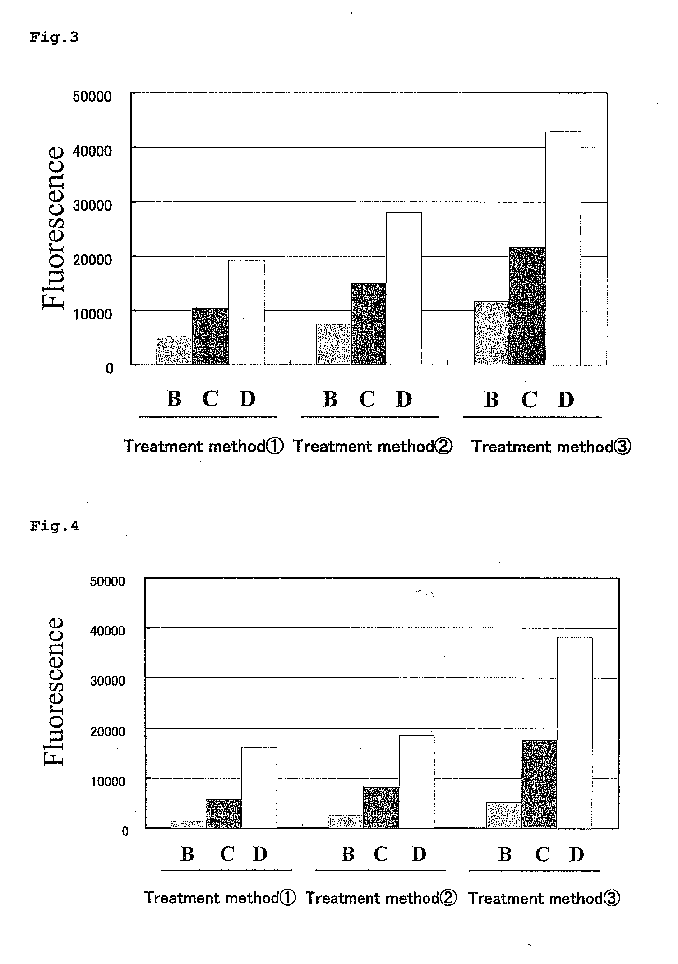

Fig. 3 is a figure showing a result of an experiment for detecting a test oligonucleotide by conducting Treatment method 1 (using only the first oligonucleotide), Treatment method 2 (using the first oligonucleotide and the second oligonucleotide) and Treatment method 3 (using the first oligonucleotide, the second oligonucleotide and the third oligonucleotide) in Example 3. In the figure, B represents a value of fluorescence measured for a test oligonucleotide concentration of 0.003 pmol/10 μL TE buffer solution. C represents a value of fluorescence measured for a test oligonucleotide concentration of 0.01 pmol/10 μL TE buffer solution. D represents a value of fluorescence measured for a test oligonucleotide concentration of 0.03 pmol/10 μL TE buffer solution.

Fig. 4 is a figure showing a result of an experiment for detecting a test oligonucleotide by conducting Treatment method 1 (using the first oligonucleotide and the (2,1) th oligonucleotide), Treatment method 2 (using the first oligonucleotide and the (2,2)th oligonucleotide) and Treatment method 3 (using the first oligonucleotide, the (2,1) th oligonucleotide and the (2,2) th oligonucleotide) in Example 4. In the figure, B represents a value of fluorescence measured for a test oligonucleotide concentration of 0.003 pmol/10 μL TE buffer solution. C represents a value of fluorescence measured for a test oligonucleotide concentration of 0.01 pmol/10 μL TE buffer solution. D represents a value of fluorescence measured for a test oligonucleotide concentration of 0.03 pmol/10 μL TE buffer solution.

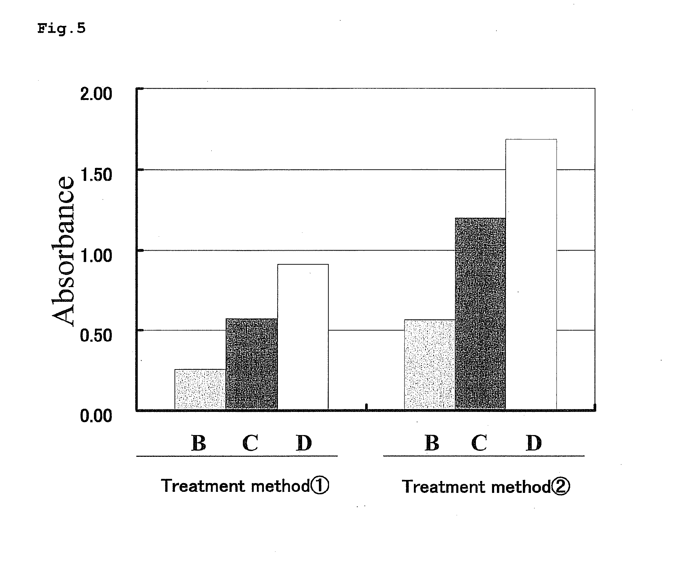

Fig. 5 is a figure showing a result of an experiment for detecting a test oligonucleotide by conducting Treatment method 1 (using the first oligonucleotide) and Treatment method 2 (using the first oligonucleotide and the second oligonucleotide) in Example 5. In the figure, B represents a value of fluorescence measured for a test oligonucleotide concentration of 0.003 pmol/10 μL TE buffer solution. C represents a value of fluorescence measured for a test oligonucleotide concentration of 0.01 pmol/10 μL TE buffer solution. D represents a value of fluorescence measured for a test oligonucleotide concentration of 0.03 pmol/10 μL TE buffer solution.

Mode for carrying out the invention

In the following, the present invention will be described in detail.

The expression "prepare a specimen containing a test oligonucleotide that is DNA comprising a target DNA region" in the first step of the present method means preparing DNA comprising a target DNA region as a DNA sample.

As the "specimen" in the present invention, for example, surface adhered matters from foods, rivers, soils or general commercial products can be recited, and these specimens may contain contaminated microorganisms such as fungi, bacteria, and viruses or nucleic acids.

In foods, it is important to inspect whether there is a food poisoning bacterium and to identify a causative bacterium, and an immunological method using an antigen of the microbial surface is usually used. However, the immunological method requires a huge amount of labor to prepare an antigen, and further requires identification of a pathogenic bacterium. In other words, since an immunological method in a microbial inspection utilizes specificity of a microbial species, not only it is difficult to examine presence or absence of a plural kinds of bacteria in one inspection, but also a huge amount of labor is required for inspection such that a PCR method or the like is used for a microorganism for which an immunological method cannot be established. The present invention is able to establish an inspection method from gene even when inspection by an immunological method is difficult, and in turn able to provide an inspection method capable of concurrently detecting a plurality of microorganisms. In other words, the present invention can be used for inspection of fungi, microorganisms, virus and the like existing in a non-biological specimen. Also by using the present method, it becomes possible to detect a contaminated microorganism or virus, for example, in food, and application in an examination of infection, a food contamination inspection or the like is expected.

As the specimen, (a) blood, a body fluid, excreta, a body secretion, a cell lysate or a tissue lysate derived from a mammal, (b) DNA extracted from one selected from the group consisting of blood, a body fluid, excreta, a body secretion, a cell lysate and tissue lysate derived from a mammal, (c) DNA prepared from RNA extracted from the one selected from the group consisting of a tissue, a cell, a tissue lysate and a cell lysate derived from a mammal as a template, (d) DNA extracted from a bacterium, a fungus or a virus, (e) DNA prepared from RNA extracted from a bacterium, a fungus or a virus as a template, and the like are recited. The term tissue is used in a broad sense including blood, lymph node and the like, and as the body fluid, plasma, serum, lymph and the like are recited, and as the body secretion, urine, milk and the like are recited.

As the DNA contained in the specimen, genomic DNA obtained by extraction from said biological sample or said contaminated microorganism, a DNA fragment derived from genomic DNA, or RNA can be recited. When the specimen derived from a mammal is human blood, a body fluid, a body secretion or the like, a sample collected in a clinical examination in a regular health examination of human or the like may be used. When blood is used as a specimen, by preparing plasma or serum according to a usual method from blood, and analyzing free DNA (containing DNA derived from a cancer cell such as a gastric cancer cell) contained in the prepared plasma or serum as a specimen, DNA derived from a cancer cell such as a gastric cancer cell can be analyzed avoiding DNA derived from blood cells and sensitivity of detecting a cancer cell such as a gastric cancer cell, a tissue containing the same and the like can be improved.

For obtaining genomic DNA from a specimen derived from a mammal, for example, a commercially available DNA extracting kit and the like may be used. For obtaining DNA from RNA, a kit for synthesizing DNA from RNA using a reverse transcriptase such as a commercially available cDNA preparation kit may be used. In the present method, the specimen may be DNA that is artificially synthesized.

The term "mammal" in the present method means animals classified into animal kingdom, Chordata, Chordate subphylum, Mammalia, and concrete examples include human being, monkey, marmoset, guinea pig, rat, mouse, bovine, sheep, dog, cat and the like.

The term "body fluid" in the present invention means a liquid existing between cells constituting an individual body, and concretely, plasma and interstitial fluid are recited, and it often functions to maintain homeostasis of an individual body. More concretely, lymph, tissue fluids (intercellular fluid, intercellular fluid, interstitial fluid), celomic fluid, serous cavity fluid, pleural effusion, ascetic fluid, pericardial fluid, cerebral fluid (spinal fluid), joint fluid (spinal fluid), eye aqueous fluid (aqueous fluid), cerebrospinal fluid, and the like are recited.

The term "body secretion" in the present invention is a secretion from an exocrine gland. Concrete examples include saliva, gastric juice, bile, pancreatic juice, intestinal juice, sweat, tear, runny nose, semen, vaginal lubricant, amniotic fluid, milk, and the like.

The "cell lysate" in the present invention means a lysate containing an intracellular fluid obtained by breaking cells cultured in a 10 cm plate for cell culture, namely, cell strains or primary cultured cells, blood cells and the like. As a method of breaking cell membranes, a method based on sonication, a method using a surfactant, a method using an alkaline solution and the like are recited. For lysing cells, a variety of kits and the like may be used.

Concretely, for example, after culturing cells to be confluent in a 10 cm plate, the culture solution is removed, and 0.6 mL of a RIPA buffer (1x TBS, 1% nonidet P-40, 0.5% sodium deoxysholate, 0.1% SDS, 0.004% sodium azide) is added to the plate. After shaking slowly the plate at 4°C for 15 minutes, cells adhered on the 10 cm plate are removed by using a scraper or the like, and the lysate liquid on the plate is transferred to a microtube. After adding 10 mg/mL PMSF in an amount of 1/10 volume of the lysate liquid, the tube is left still on ice for 30 to 60 minutes. Centrifugation at 10,000xg is conducted at 4°C for 10 minutes, to obtain the supernatant as a cell lysate.

The "tissue lysate" in the present invention means a lysate containing an intracellular fluid obtained by breaking cells in tissues collected from an animal such as a mammal.

More concretely, after measuring weight of the tissue obtained from an animal, the tissue is cut into small pieces with the use of a razor or the like. When a frozen tissue is sliced, it is necessary to make a smaller piece. After cutting, an ice-cooled RIPA buffer (protease inhibitor, phosphatase inhibitor and the like may be added, and for example, 10 mg/mL PMSF in an amount of 1/10 volume of the RIPA buffer may be added) is added in a rate of 3 mL per 1 g of tissue, and homogenized at 4°C. For homogenization, a sonicator or a pressurized cell grinder is used. In an operation of homogenization, the solution is constantly kept at 4°C for preventing heat generation. The homogenized liquid is transferred to a microtube, and centrifuged at 4°C for 10 minutes at 10,000xg, and the supernatant is obtained as a tissue lysate.

The first step is a step of preparing a specimen containing a test oligonucleotide that is DNA comprising a target DNA region.

The "test oligonucleotide" in the present invention is an oligonucleotide containing a target DNA region. Further, the test oligonucleotide may be DNA containing a target region, or may be RNA containing a target region. Concrete examples include a target DNA region in genomic DNA, target RNA (or a part thereof), or DNA (or a part thereof) prepared from RNA as a template, or a DNA fragment or a RNA fragment containing the same. The test oligonucleotide in the present method may be artificially synthesized.

In the present invention, for example, when the test oligonucleotide is DNA extracted from a bacterium, fungus or virus, RNA extracted from a bacterium, fungus or virus or DNA that is prepared from the RNA as a template, and an oligonucleotide comprising a partial sequence thereof, detection of the test oligonucleotide means quantification or detection of an index that allows estimation of presence or absence and the kind of a microorganism or a virus causing the microbial contamination in food or infection. Further, for example, when the test oligonucleotide represents free DNA in blood and an oligonucleotide comprising its partial sequence, detection of the test oligonucleotide means quantification or detection of an index that allows estimation of degree of progression of cancer by quantification of free DNA in blood. When the test oligonucleotide represents DNA or RNA extracted from a tissue, tissue lysate, cell lysate or tissue lysate, or DNA prepared from the RNA as a template, and an oligonucleotide comprising a partial sequence of these, detection of the test oligonucleotide means quantification of an amount of RNA functioning in a cell, and quantification or detection of an index that allows estimation of a function, property and condition of the cell related with the function of the RNA. For example, if RNA or DNA originating from a microorganism or a virus is detected from a tissue lysate, tissue infection by a microorganism or a virus can be estimated. For example, in cancer, it is known that free DNA originating from genomic DNA in blood increases, and quantification or detection of free DNA in blood provides an index allowing estimation of the degree of progression of the cancer (condition). In this case, as the test oligonucleotide, any of free DNA derived from genomic DNA in blood or an oligonucleotide containing a target DNA region contained in the free DNA, and an oligonucleotide comprising a partial sequence of these is preferred.

The expression "prepare a specimen containing a test oligonucleotide that is DNA comprising a target DNA region" in the first step includes obtaining a DNA sample containing a target DNA region intended to be detected, as a test oligonucleotide. For example, when a nucleotide sequence on RNA is intended to be detected, DNA that has been synthesized by a reverse transcriptase from the RNA as a template is obtained as a test oligonucleotide containing a complementary nucleotide sequence of the nucleotide sequence intended to be detected on the RNA as a target DNA region. When RNA is intended to be detected, the expression "obtain a test oligonucleotide that is RNA comprising a target region from a specimen" means obtaining a RNA sample containing a target RNA region intended to be detected as a test oligonucleotide.

When the test oligonucleotide obtained in the first step is DNA, it may be digested in advance with a restriction enzyme recognition cleavage site for which is not present in the target region possessed by the test oligonucleotide, or may be contained in a DNA sample that has been purified in advance. Besides, as DNA obtained in the first step, free DNA in blood, DNA derived from microbial genome, DNA prepared by a reverse transcriptase from RNA in a specimen and the like are recited. Further, the "DNA comprising a target DNA region" may be synthesized DNA. When the test oligonucleotide obtained in the first step is RNA, free RNA of a tissue lysate or cell lysate, purified RNA, RNA obtained from a microorganism and the like are recited.

In the first step, for obtaining genomic DNA containing a nucleotide sequence of a target DNA region, for example, a commercially available DNA extraction kit (Genfind v2 Kit (available from BECKMAN COULTER, Inc.), FastPure DNA Kit (available from TAKARA BIO INC.)) and the like may be used, when the specimen is derived from a mammal. When the specimen is a microorganism such as fungus, a general preparation method of yeast genome or the like as described in Methods in Yeast Genetics (Cold Spring Harbor Laboratory Press) may be used, while when the specimen is a prokaryote such as Escherichia coli, a general preparation method of microorganism genome or the like as described in Molecular Cloning -A Laboratory Manual- (Cold Spring Harbor Laboratory Press) may be used.

When RNA is intended to be obtained from a specimen such as a tissue or cell strain derived from a mammal, RNA may be extracted from a tissue, cell strain and the like using a commercially available RNA extraction kit (ISOGEN (311-02501) (available from NIPPON GENE CO., LTD.), or FastRNA Pro Green Kit (available from Funakoshi Corporation), FastRNA Pro Blue Kit (available from Funakoshi Corporation), FastRNA Pro Red Kit (available from Funakoshi Corporation), and the like). For synthesizing DNA using RNA as a template, a reverse transcriptase may be used, and a commercially available kit (transcriptor high fidelity cDNA synthesis kit, available from Roche Diagnostics K.K.) may be used. Viral DNA may be extracted after extracting viral particles. Viral genome may be extracted using a commercially available kit (QuickGene RNA tissue kit SII, available FUJIFILM Corporation) or the like. Alternatively, DNA originating from a virus may be obtained by a reverse transcriptase from RNA extracted from a tissue infected by a virus, or DNA may be obtained from a tissue infected by a virus.

When the specimen is food, DNA may be prepared after separating a microorganism or the like from the food, and genomic DNA derived from other organism than microorganisms such as virus, and genomic DNA derived from a microorganism contained in the food may be obtained concurrently.

When the specimen is a tissue derived from a mammal, and the target DNA region is DNA derived from a virus, RNA may be extracted from the tissue using such as a commercially available RNA extraction kit (ISOGEN(311-02501) (available from NIPPON GENE CO., LTD.), or FastRNA Pro Green Kit (available from Funakoshi Corporation), FastRNA Pro Blue Kit (available from Funakoshi Corporation), FastRNA Pro Red Kit (available from Funakoshi Corporation), and the like), and DNA may be obtained by a reverse transcriptase. When the specimen is a specimen derived from a mammal, viral DNA may be extracted after extracting virus particles, or after extracting virus particles, viral DNA may be extracted using a commercially available kit (QuickGene RNA tissue kit SII, available FUJIFILM Corporation) or the like, and DNA derived from the virus may be obtained by a reverse transcriptase. RNA may be extracted from a tissue infected by a virus, and DNA derived from the virus may be obtained by a reverse transcriptase, or DNA may be obtained from a tissue infected by a virus, and DNA derived from the virus may be obtained. When DNA is obtained from RNA by a reverse transcriptase, a commercially available kit (transcriptor high fidelity cDNA synthesis kit, available from Roche Diagnostics K.K.) may be used.

The "target DNA region" (hereinafter, also described as a target region) in the present invention means a DNA region intended to be detected or quantified by the present invention among DNA contained in a specimen. When the specimen is DNA, the target DNA region is a predetermined nucleotide sequence on a nucleotide sequence of the DNA, and when the specimen is RNA, it is a nucleotide sequence on DNA prepared from RNA by a reverse transcriptase, and is a complementary nucleotide sequence of a predetermined nucleotide sequence intended to be detected or quantified on the RNA. The "DNA comprising a target DNA region" may be DNA that is digested in adbance with a restriction enzyme not having its recognition cleavage site in the nucleotide sequence in the target DNA region possessed by the DNA, or may be a DNA sample purified in advance, free DNA in blood, DNA derived from microbial genome, DNA synthesized from RNA in a specimen by a reverse transcriptase and the like.

The "target DNA region" may be a nucleotide sequence found plurally in genome (hereinafter, also referred to as a repetitive sequence), and a nucleotide sequence that will be an index for a disease is more preferred. For example, in cancer, it is known that free DNA derived from genomic DNA in blood increases, and in this case, as the test oligonucleotide, oligonucleotides comprising a repetitive sequence in free DNA in blood and its partial sequence are recited. The quantified value of such an oligonucleotide comprising a repetitive sequence or its partial sequence can be regarded as an index representing the degree of progression of the cancer. As will be described later, the repetitive sequence may be a simple repetitive sequence (called a tandem repetitive sequence or a tandem repeat), an interspersed repetitive sequence, a duplicate gene, a pseudo gene and the like.

The "target RNA region" in the present invention means, in one sense, a nucleotide sequence intended to be detected on RNA in a biological cell. As "RNA comprising a target RNA region" contained in a biological specimen, RNA extracted from a specimen is recited, and concretely, ribosomal RNA, messenger RNA, transfer RNA, and micro RNA and the like are recited. As the RNA, not only the one that has been transcribed from genome of a host by RNA polymerase, but also the one containing genomic RNA of a virus whose genome is RNA are included, and any RNA is applicable.

The "target DNA region" in the present invention may be a gene related with a disease or the like. For example, as genes related with cancer, promoter regions, untranslated regions or translated regions (coding regions) and the like of genes of useful proteins such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin2, Neurofilament3, disintegrin and metalloproteinase domain 23, G protein-coupled receptor 7, G-protein coupled somatostatin and angiotensin-like peptide receptor, and Solute carrier family 6 neurotransmitter transporter noradrenalin member 2 can be recited. In said "target DNA region", methylated DNA may be detected or quantified individually, and, for example, when more "target DNA region" is detected or quantified in one detection system, the quantification accuracy and detection sensitivity are improved correspondingly.

To be more specific, when the useful protein gene is a Lysyl oxidase gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, a nucleotide sequence of Lysyl oxidase gene (corresponding to the nucleotide sequence represented by base No. 16001 to 18661 in the nucleotide sequence described in Genbank Accession No. AF270645) can be recited. In this region, ATG codon encoding methionine at amino terminal of Lysyl oxidase protein derived from human is represented in base No. 18031 to 18033, and a nucleotide sequence of the above exon 1 is represented in base No. 17958 to 18662.

To be more specific, when the useful protein gene is a HRAS-like suppressor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, a nucleotide sequence of HRAS-like suppressor gene (corresponding to the nucleotide sequence represented by base No. 172001 to 173953 in the nucleotide sequence described in Genbank Accession No. AC068162) can be recited. In this region, the nucleotide sequence of exon 1 of a HRAS-like suppressor gene derived from human is represented in base No. 173744 to 173954.

To be more specific, when the useful protein gene is a bA305P22.2.1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, a nucleotide sequence of bA305P22.2.1 gene (corresponding to the nucleotide sequence represented by base No. 13001 to 13889 in the nucleotide sequence described in Genbank Accession No. AL121673) can be recited. In this region, ATG codon encoding methionine at amino terminal of bA305P22.2.1 protein derived from human is represented in base No. 13850 to 13852, and a nucleotide sequence of the above exon 1 is represented in base No. 13664 to 13890.

To be more specific, when the useful protein gene is a Gamma filamin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, a nucleotide sequence of Gamma filamin gene (corresponding to a complementary sequence to the nucleotide sequence represented by base No. 63528 to 64390 in the nucleotide sequence described in Genbank Accession No. AC074373) can be recited. In this regoin, ATG codon encoding methionine at amino terminal of Gamma filamin protein derived from human is represented in base No. 64101 to 64103, and a nucleotide sequence of the above exon 1 is represented in base No. 63991 to 64391.

To be more specific, when the useful protein gene is a HAND1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, a nucleotide sequence of HAND1 gene (corresponding to a complementary sequence to the nucleotide sequence represented by base No. 24303 to 26500 in the nucleotide sequence described in Genbank Accession No. AC026688) can be recited. In this region, ATG codon encoding methionine at amino terminal of HAND1 protein derived from human is represented in base No. 25959 to 25961, and a nucleotide sequence of the above exon 1 is represented in base No. 25703 to 26501.

To be more specific, when the useful protein gene is a Homologue of RIKEN 2210016F16 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, a nucleotide sequence of Homologue of RIKEN 2210016F16 gene (corresponding to a complementary nucleotide sequence to the nucleotide sequence represented by base No. 157056 to 159000 in the nucleotide sequence described in Genbank Accession No. AL354733) can be recited. In this region, a nucleotide sequence of exon 1 of a Homologue of a RIKEN 2210016F16 gene derived from human is represented in base No. 158448 to 159001.

To be more specific, when the useful protein gene is a FLJ32130 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, a nucleotide sequence of FLJ32130 gene (corresponding to a complementary nucleotide sequence to the nucleotide sequence represented by base No. 1 to 2379 in the nucleotide sequence described in Genbank Accession No. AC002310) can be recited. In this region, ATG codon encoding methionine at amino terminal of FLJ32130 protein derived from human is represented in base No. 2136 to 2138, and a nucleotide sequence assumed to be the above exon 1 is represented in base No. 2136 to 2379.

To be more specific, when the useful protein gene is a PPARG angiopoietin-related protein gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, a nucleotide sequence of SEQ ID NO: 8 can be recited. In this region, preferable is a region containing about 1200 bases to 2000 bases of 5'-side part of ATG codon encoding methionine at amino terminal of PPARG angiopoietin-related protein derived from human.

To be more specific, when the useful protein gene is a Thrombomodulin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, a nucleotide sequence of Thrombomodulin gene (corresponding to the nucleotide sequence represented by base No. 1 to 6096 in the nucleotide sequence described in Genbank Accession No. AF495471) can be recited. In this region, ATG codon encoding methionine at amino terminal of Thrombomodulin protein derived from human is represented in base No. 2590 to 2592, and a nucleotide sequence of the above exon 1 is represented in base No. 2048 to 6096.

To be more specific, when the useful protein gene is a p53-responsive gene 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, a nucleotide sequence of p53-responsive gene 2 gene (corresponding to a complementary sequence to the nucleotide sequence represented by base No. 113501 to 116000 in the nucleotide sequence described in Genbank Accession No. AC009471) can be recited. In this region, a nucleotide sequence of exon 1 of a p53-responsive gene 2 gene derived from human is represented in base No. 114059 to 115309.

To be more specific, when the useful protein gene is a Fibrillin2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, a nucleotide sequence of Fibrillin2 gene (corresponding to a complementary sequence to a nucleotide sequence represented by base No. 118801 to 121000 in the nucleotide sequence described in Genbank Accession No. AC113387) can be recited. In this region, a nucleotide sequence of exon 1 of a Fibrillin2 gene derived from human is represented in base No. 119892 to 112146.

To be more specific, when the useful protein gene is a Neurofilament3 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, a nucleotide sequence of Neurofilament3 gene (corresponding to a complementary sequence to a nucleotide sequence represented by base No. 28001 to 30000 in the nucleotide sequence described in Genbank Accession No. AF106564) can be recited. In this region, a nucleotide sequence of exon 1 of a Neurofilament3 gene derived from human is represented in base No. 28615 to 29695.

To be more specific, when the useful protein gene is a disintegrin and metalloproteinase domain 23 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, a nucleotide sequence of disintegrin and metalloproteinase domain 23 gene (corresponding to the nucleotide sequence represented by base No. 21001 to 23300 in the nucleotide sequence described in Genbank Accession No. AC009225) can be recited. In this region, a nucleotide sequence of exon 1 of a disintegrin and metalloproteinase domain 23 gene derived from human is represented in base No. 22195 to 22631.

To be more specific, when the useful protein gene is a G protein-coupled receptor 7 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, a nucleotide sequence of G protein-coupled receptor 7 gene (corresponding to a nucleotide sequence represented by base No. 75001 to 78000 in the nucleotide sequence described in Genbank Accession No. AC009800) can be recited. In this region, a nucleotide sequence of exon 1 of a G protein-coupled receptor 7 gene derived from human is represented in base No. 76667 to 77653.

To be more specific, when the useful protein gene is a G-protein coupled somatostatin and angiotensin-like peptide receptor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, a nucleotide sequence of G-protein coupled somatostatin and angiotensin-like peptide receptor gene (corresponding to a complementary sequence to the nucleotide sequence represented by base No. 57001 to 60000 in the nucleotide sequence described in Genbank Accession No. AC008971) can be recited. In this region, a nucleotide sequence of exon 1 of a G-protein coupled somatostatin and angiotensin-like peptide receptor gene derived from human is represented in base No. 57777 to 59633.

To be more specific, when the useful protein gene is a Solute carrier family 6 neurotransmitter transporter noradrenalin member 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Solute carrier family 6 neurotransmitter transporter noradrenalin member 2 gene derived from human, and a promoter region located 5' upstream of the same can be recited, and more concretely, a nucleotide sequence of Solute carrier family 6 neurotransmitter transporter noradrenalin member 2 gene (corresponding to a complementary sequence to the nucleotide sequence represented by base No. 78801 to 81000 in the nucleotide sequence described in Genbank Accession No. AC026802) can be recited. In this region, a nucleotide sequence of exon 1 of a Solute carrier family 6 neurotransmitter transporter noradrenalin member 2 gene derived from human is represented in base No. 80280 to 80605.

As the "target DNA region" (hereinafter, also referred to as a target region), for example, regions of DNA containing a nucleotide sequence of a promoter region, untranslated region or translated region (coding region) of gene represented by the symbols of MLH1, RUNX3, CDH1, TIMP3, CSPG, RARβ, 14-3-3σ, CALCA, HIC1, ESR1, PTEN, SOCS1, BLT1, ESR2, MTMG, TWIST, INK4, CDKN2, GSTP, DCR2, TP73, PGR, HIC2, MTHFR, TFF1, MLLT7, SLC5A8, THBS1, SOCS2, ACTB, CDH13, FGF18, GSTM3, HSD17B4, HSPA2, PPP1R13B, PTGS2, SYK, TERT, TITF1, BRACA1, AATF, ABCB1, ABCC1, ABI1, ABL1, AF1Q, AF3P21, AF4, AF9, AFF3, AKAP12, AKAP9, ALEX3, ALK, ALOX15, APAF1, APC, ARHA, ARHGAP26, ARHGEF12, ARNT, ATBF1, ATF1, ATM, AXIN2, BCAS3, BCAS4, BCL1, BCL10, BCL11A, BCL11B, BCL2, BCL5, BCL7A, BCR, BIRC3, BMPR1A, BRCA2, BRD4, BRIP1, BTG1, BUB1B, CAGE-1, CARS, CASC5, CCDC6, CCND2, CCND3, CDH11, CDKN1B, CDKN2A, CDX2, CEP110, CKN1, CLP1, CLTC, CLTCL1, CNC, COL1A1, COX6C, CREBBP, CXXC6, DAB2IP, DDIT3, DDX43, DIRC1, IRC2, DKK1, E2F3, EEN, EGFR, ELL, EPS15, ERBB2, ERC1, ERCC1, ERCC4, ERG, ETV1, ETV6, EVI1, EWSR1, EXT1, EXT2, FANCA, FANCD2, FANCF, FAS, BP17, FCRL4, FEV, FGFR1, FHIT, FLI1, FOX03A, FUS, FVT1, GAS7, GLI1, GNAS, GOLGA5, GOPC, GRB2, HCMOGT-1, HIST1H4I, HLF, HMGA2, HOXA13, HOXC11, HOXC13, HOXD13, HSPBAP1, HSPCB, HSPD1, HSPH1, IKZF2, INTS6, IRF4, JAG1, JAG2, JAK2, JARID1A, JAZF1, JMJD2C, JUN, KIT, KITLG, KLF5, KLF6, LASP1, LDB1, LHFP, LM01, LMO2, LPHN2, LPP, LYL1, MADH4, MAF, MAFB, MDM2, MDS2, MET, MKL1, MLF1, MLH1, MLL, MLLT10, MMP2, MN1, MRE11B, MSF, MSH2, MSH6, MSI2, MUC1, MUTYH, MXI1, MYC, MYH9, MYST3, NF1, NFKB1, NIN, NKX2-5, NONO, NOTCH1, N-RAS, NTRK3, NUMA1, NUP214, NUP98, OLIG2, P53, PALB2, PAX2, PAX5, PAX7, PAX9, PBX1, PCM1, PCSK7, PDGFB, PDGFRA, PDGFRB, PHOX2B, PICALM, PLAG1, PLCB1, PLK, PML, PMS1, POLH, POU5F1P1, POU6F2, PPP1R13L, PRDM16, PRRX1, PSIP1, PTCH, RABEP1, RAD51L1, RAD53, RANBP17, RAP1GDS1, RAP2B, RARA, RASSF1, RB1, RBL2, RBM15, RBM5, RCHY1, RECQL, RECQL5, RET, RUNX, RUNX1T1, SBDS, SDHC, SDHD, SET, SHH, SIL, SIX1, SNAI2, SPI1, SPINK7, STARD3, STAT3, STK11, STK4, SUFU, SYK, SYNP02, TBX2, TCF3, THBS2, THRAP3, TMPRSS2, TNF, TOP1, TPM4, TPR, TRIM24, TRIM33, TRIP11, TSC1, TSC2, TSHR, TTL, VAV1, VHL, WFDC1, WT1, WWOX, XPC, ZBTB16, ZNF146, ZNF217 and the like can be recited. In the method of the present invention, these target DNA regions may be quantified or detected individually, however, when more "target DNA regions" are quantified or detected in one detection system, the quantification accuracy and detection sensitivity are improved correspondingly.

When the target region in the present invention is a nucleotide sequence derived from a microorganism, as the target region, genomic DNA or a DNA fragment extracted from a specimen, or a nucleotide sequence of DNA prepared by a reverse transcriptase from RNA extracted from a specimen can be recited. Therefore, as a nucleotide sequence capable of complementarily binding with the detection oligonucleotide, a region specific for the microorganism may be selected. For example, when the target region in the present invention is a microbial nucleotide sequence, for selectively extracting the target region from a specimen, a nucleotide sequence characteristic to the microorganism located near the target region may be selected as a nucleotide sequence specifically binding with the specific oligonucleotide, among nucleotide sequences of microbial genomic DNA, DNA prepared from RNA extracted from a specimen by a reverse transcriptase and the like.

Examples of the "DNA comprising a target DNA region" in the present method include DNA derived from microorganisms such as gram-positive bacteria, gram-negative bacteria, fungi, viruses and pathogenic protozoans, and DNA obtained from RNA derived from such microorganisms by a reverse transcriptase. For example, genomic DNA or DNA prepared by a reverse transcriptase from RNA of Mycoplasma genitalium, Mycoplasma pneumoniae, Borrelia burgdorferi B31, Rickettsia prowazekii, Treponema pallidum, Chlamydia pneumoniae, Chlamydia trachomatis, Helicobacter pylori J99, Helicobacter pylori 26695, Haemophilus influenzae Rd, Mycobacterium tuberculosis H37Rv, Pseudomonas aeruginosa, Legionella pneumophila, Serratia marcescens, Escherichia coli, Listeria monocytogenes, Salmonella enterica, Campylobacter jejuni subsp. Jejuni, Staphylococcus aureus, Vibrio parahaemolyticus, Bacillusu cereus, Clostridium botulinum, Clostridium perfringens, Yersinia enterocolitica, Yersinia pseudotuberuculosis, Trichophyton ruburum, Trichophyton mentagrophytes, Candida albicans, Cryptococcus neoformans, Aspergillus fumigatus, Pneumocystis carinii, Coccidioides immitis, Cytomegalovirus, human herpesvirus 5, Epstein-Barr virus, Human Immunodeficiency Virus, Human Papilloma Virus, Enterovirus, Norovirus Influenza Virus, Toxoplasma gondii, Cryptosporidium parvum, or Entamoeba histolytica may be used for detection of a microorganism responsible for an infection in a specimen, or a microorganism responsible for a food poisoning in food.

Generally, for examining presence or absence of a pathogenic microorganism contained in a biopsy sample or in general products such as food, presence or absence of such a pathogenic microorganism is examined, or such a pathogenic microorganism is identified by a test based on immunization method for an antigen of each microorganism or the like. However, preparation of an antibody used for a test based on an immunization method is sometimes not easy, and for detecting a plurality of bacteria, it is necessary to prepare antibodies against antigens of individual bacteria or the like. By using the present invention, it is possible to conduct the test without preparing an antibody. That is, by using the present invention, it is possible to provide a simple test method for a microorganism for which preparation of an antibody is difficult, or for a microorganism for which an antibody is not prepared. Also in the present invention, since nucleotide sequences of different microorganisms can be tested concurrently, it becomes possible to detect several kinds of microorganisms contained in one specimen concurrently. As such a microorganism, concretely, Listeria monocytogenes, Salmonella enterica, Campylobacter jejuni subsp. Jejuni, Staphylococcus aureus, Vibrio parahaemolyticus, Bacillusu cereus, Clostridium botulinum, Yersinia enterocolitica, Yersinia pseudotuberuculosis, Clostridium perfringens and the like food poisoning bacteria are known, however, a technique of concurrently detecting several kinds of these bacteria is not known. In the present invention, it is possible to test presence or absence of a plurality of food poisoning bacteria concurrently since a plurality of nucleotide sequences can be detected at the same time. Further, when a nucleotide sequence found plurally in genome such as CRISPR (Clustered regularly interspaced short palindromic repeats) region is selected (bound) as a nucleotide sequence to be detected by a specific oligonucleotide as will be described later, higher detection sensitivity is realized compared to the case of detecting one gene in one genome. Such a technique is useful also for diagnosis of infection and rapid detection of food poisoning bacteria. Further, the present invention may be used for identification of an industrially useful bacterium, or for a simple test of a microbial community in soil, river or lake sediments and the like by detecting genomic DNA of microorganisms in such environments. Of the microorganisms in such environments, inhabitation of, for example, Methanococcus jannaschii, Methanobacterium thermoautotrophicum deltaH, Aquifex aeolicus, Pyrococcus horikoshii OT3, Archaeoglobus fulgidus, Thermotoga maritima MSB8, Aeropyrum pernix K1, Haloferax mediterranei and the like can be verified. It is also possible to detect and identify industrially available bacteria such as Geobacter sulfurreducens and microorganisms used for fermentation such as Streptococcus thermophilus.

For example, as a region where the target region for detecting genome in a microorganism and the detection oligonucleotide as will be described later complementarily bind, concretely, the nucleotide sequence of the base numbers 271743 to 272083 of yeast chromosome VII as shown, for example, in Genbank Accession No. NC_001139, or a nucleotide sequence not encoding a gene such as the nucleotide sequence of the nucleotide numbers 384569 to 384685 of yeast chromosome VII shown, for example, in Genbank Accession No. NC_001139 is applicable. It is also useful to detect a nucleotide sequence conserved among pathogenic microorganisms in a characteristic gene that is common in various pathogenic microorganisms to provide a method of concurrently detecting a plurality of pathogenic microorganisms. Concretely, since mce-family gene (Micobacterium tuberculosis), tRNA-Tyr nucleotide sequence on 13th chromosome (Cryptococcus neoformans), and chitin synthase activator (Chs3) have a nucleotide sequence peculiar to Aspergillus fumigatus and genus Neosartorya, they can be used for assay of infection by a microorganism, by assaying whether DNA originating from these microorganisms is contained in DNA extracted from a biopsy sample of human expectoration or lung. Further, since actA (Listeria monocytogenes), pyrG (NC_002163, Campylobacter jejuni subsp. jejuni) and the like are common genes peculiar to food poisoning bacteria, these genes may be used for a microbial assay in food poisoning. Also, thrA has a sequence that is conserved among Salmonella enterica, Yersinia enterocolitica, and Escherichia coli, so that a plurality of microorganisms can be detected by one gene.

The "repetitive sequence" in the present method means a nucleotide sequence for which the identical predetermined sequence is plurally found in genome. As such a repetitive sequence, a simple repetitive sequence (called a tandem repetitive sequence or a tandem repeat), an interspersed repetitive sequence and the like are known.

The simple repetitive sequence is characterized in that the identical sequences neighbor in the same orientation, and a series of nucleotide sequences such as satellite DNA, minisatellite, microsatellite, centromere, telomere, kinetochore, and ribosome group genes are known.

The interspersed repetitive sequence is characterized in that the identical sequences are interspersed without neighboring each other, and is believed to be DNA derived from retrotransposon. Interspersed repetitive sequences are classified into SINE (Short Interspersed Repetitive Element: short chain interspersed repetitive sequence) and LINE (Long Interspersed Elements:long chain interspersed repetitive sequence) depending on the length of the nucleotide sequence, and Alu sequence and LINE-1 sequence are respectively known as representative repetitive sequences as human nucleotide sequences. Also an inactive processed pseudo gene that is counter-transferred from RNA or protein, and a gene sequence amplified by gene duplication are known.

The term duplicate gene indicates the case that a plurality of genes having high homology exist on one genome, and in many cases, it includes nucleotide sequences existing in tandem near one gene. Some pseudo genes are known to be included in duplicate genes.

As concrete examples of the repetitive sequence, such sequences as (A)n, (T)n, (GA)n, (CA)n, (TAA)n, (GGA)n, (CAGC)n, (CATA)n, (GAAA)n, (TATG)n, (TTTG)n, (TTTA)n, (TTTC)n, (TAAA)n, (TTCA)n, (TATAA)n, (TCTCC)n, (TTTCC)n, (TTTAA)n, (TTTTC)n, (TTTTA)n, (TTTTG)n, (CAAAA)n, (CACCC)n, (TATATG)n, (CATATA)n, (TCTCTG)n, (AGGGGG)n, (CCCCCA)n, and (TGGGGG)n (n means a number of repetition) are known as repetition comprising a relatively short nucleotide sequence, and as a sequence derived from a transcription factor, MER1-Charlie, and Zaphod of hAT group, and MER2-Tigger, Tc-1, and Mariner of Tc-1 group can be recited. As others, concretely, Tigger1, Tigger2a, Tigger5, Charlie4a, Charlie7 and the like are known. These sequences are generally short and simple nucleotide sequences, and are difficult to set the specific adhesion sequence as will be described later, however, these sequences can be used in the present invention as far as they have a sequence that can be set into setting objects of the specific adhesion sequence and a detection adhesion sequence as will be described later. Therefore, it is not necessarily excluded as an object of the present invention. Further, satellite DNA, minisatellite, microsatellite and the like are repetitive sequences classified into simple repetitive sequences.

Further, as a sequence having multi-copies in gene, ALR6 as a sequence existing in centromere, U2 and U6 as snRNA, as well as the genes such as tRNA and rRNA that are generally known to have multi-copies in genome, and the genes that have plural copies in genome as a result of gene duplication are recited.

A pseudogene means a gene having a characteristic nucleotide sequence that is assumable to have encoded a gene product (particularly protein) in a sequence of DNA, but currently loosing the function. It is assumed that it is generated as a result of mutation of the original functioning sequence. For example, there is the case where a stop codon arises by mutation and a peptide chain of a protein is shortened, so that the function as a protein is no longer effective, and there is the case where a function of a regulatory sequence required for normal transcription is impaired due to mutation such as single nucleotide substitution. In many pseudogenes, the original normal genes are remained separately, however, those becoming pseudogenes by themselves are also known.

Pseudogenes are classified into three types according to the characteristic of the gene sequence. There are known the case where DNA prepared from mRNA by a reverse transcriptase of retrotransposon is inserted into genome (processed pseudogene), the case where an original gene sequence is duplicated in genome, and a part of the copies looses the function due to mutation or the like to become a pseudogene (duplicated pseudogene or non-processed pseudogene), and the case where gene in genome (in the condition of single gene with no duplicated gene) looses the function to become a pseudogene.

Currently, among the genes known as pseudogenes, transcribed examples, examples having a gene function (whether it is called a pseudogene is not determined) and the like also have been known, so that the term "pseudogene" in the present method means the "processed pseudogene" or "duplicated pseudogene (non-processed pseudogene)" rather than presence or absence of gene function or whether it is transcribed or not.

The term duplicate gene means a gene or a gene fragment that is generated by doubling of a specific gene or gene fragment in genome due to gene duplication. Gene duplication is a phenomenon that a region of DNA including a gene is overlapped. As a cause of gene duplication, abnormality of gene recombination, translocation of retrotransposon, duplication of the entire chromosome and the like are recited. For example, when one gene is copied and inserted into genomic DNA, it is inserted into a different chromosome site in one case, and inserted near the original gene in the other case. The site where the copied gene stands in line as a result of insertion near the original gene is called a tandem repeat, and a group of genes generated by gene duplication is called a gene family.

It is also known that a retrovirus, a retrotransposon having LTR (Long terminal repeat) in its terminal, an endogenous sequence such as MaLRs (Mammalian apparent LTR-Retrotransposons) considered to be derived from viruses, and LTR derived from a retrovirus exist in multicopy in one genome.

For example, as the LTR derived from a retrovirus, concretely, subfamilies such as LTR1, LTR1B, LTR5, LTR7, LTR8, LTR16A1, LTR16A1, LTR16C, LTR26, LTR26E, MER48, and MLT2CB are known. The LTRs derived from a retrotransposon are classified into classes of ERV, ERVK and ERVL, and concrete examples include subfamilies such as LTR8A, LTR28, MER21B, MER83, MER31B, MER49, MER66B, HERVH, ERVL, LTR16A1, LTR33A, LTR50, LTR52, MLT2A1, MLT2E, MER11C, and MER11C. Further, MaLRs indicate DNA factors including LTRs in both ends likewise a typical retrotransposon, wherein an internal sequence sandwiched between LTRs is not derived from a retrovirus. For example, subfamilies such as MLT1A1, MLT1A2, MLT1B, MLT1C, MLT1D, MLT1F, MLT1G, MLT1H, MLT1J, MLT1K, MLT1I, MLT2CB, MSTA, MSTA-int, MSTB, THE1A, THE1B, THE1B-internal, and THE1 can be recited.

The "interspersed repetitive sequences" are characterized by being interspersed without neighboring each other, and are considered to be derived from a retrotransposon. Further, the interspersed repetitive sequences are classified into SINE (Short Interspersed Repetitive Element: short chain interspersed repetitive sequences) and LINE (Long Interspersed Elements: long-chain interspersed repetitive sequences) according to the length. Most of SINEs are sequences belonging to the Alu family. A common feature is that it has a sequence of 3'-side or a sequence of 5'-side of 7SL RNA, and that it has an AT-Rich region sandwiched between a Left-monomer and a Right-monomer. As subfamilies of the Alu family, Alu, AluJb, AluJo, AluSc, AluSg, AluSp, AluSq, AluSx, AluY, and FAM (Fossil Alu Monomer), FLAM (Free Left Alu Monomer) having a sequence of FAM, and FRAM (Free Right Alu Monomer) can be recited. As SINEs other than the Alu family, MIR, and Ther/MIR3 are known, and MIR and MIR3 are known as respective subfamilies. As subfamilies of the Alu family including other biological species, B1, B2, B4, PB1, PB1D and so on are known. As LINES, subfamilies of LINE1 to Line23 are reported, and it is known that subfamilies such as LINE-1, LINE2, and LINE3 broadly exist in a genome. As for LINE-1, for example, L1M1, L1M2, L1M3, L1M3d, L1M4, L1M4c, L1MA2, L1MA7, L1MA8, L1MA9, L1MB1, L1MB1, L1MB3, L1MB4, L1MB5, L1MB6, L1MB7, L1MCa, L1MCb, L1MC2, L1MC3, L1MC4, L1MC4a, L1MC5, L1MDa, L1ME, L1MEc, L1MEd, L1MEg, L1ME1, L1ME2, L1ME3, L1ME3A, L1ME3B, L1ME4a, L1PB3, L1P4, L1PA2, L1PA3, L1PA4, L1PA5, L1PA6, L1PA7, L1PA10, L1PA12, L1PA13, L1PA14, L1PA16, L1PB1, L1PB3, L1PB4, L1PREC2, and HAL1 are known, and as LINE-2, subfamilies such as L2 and L2c are known. For example, if the later-described specific adhesion sequence and the detection adhesion sequence can be set, for a sequence common to the Alu family or subfamilies of Alu, or the LINE-1 family or subfamilies of LINE-1, a plurality of detection objects can be set in one genome, so that sensitivity of genome detection can be improved.

As a target DNA region, concretely, for example, a partial sequence of LINE-1 (the nucleotide sequence of SEQ ID NO: 12, SEQ ID NO: 13, or SEQ ID NO: 14), a partial sequence of Alu (the nucleotide sequence of SEQ ID NO: 15) or nucleotide sequences having homology to these sequences can be recited.

In the present invention, measuring a repetitive sequence means concurrent measurement of a nucleotide sequence existing plurally in one genome, and for example, a nucleotide sequence having a sequence homology of 80% or higher with the nucleotide sequence of SEQ ID NO: 13 has about 280 copies in a human genome, and a nucleotide sequence having a sequence homology of 80% or higher with the nucleotide sequence of SEQ ID NO: 15 has about 820 copies in a human genome. Therefore, if one can set a detection adhesion sequence and a specific adhesion sequence in each nucleotide sequence, the detection sensitivity of one genome can be improved to 280 to 820 folds theoretically, compared to the case where a detection adhesion sequence and a specific adhesion sequence are set for a sequence having just one kind in genome.

The second step is a step of mixing the test oligonucleotide contained in the specimen prepared in the first step, and a detection oligonucleotide that is capable of complementarily binding with the test oligonucleotide and has a plurality of identification functions, to form a detection complex comprising the test oligonucleotide and the detection oligonucleotide, and immobilizing the detection complex to a support.

As the detection oligonucleotide having a plurality of identification functions, a composite detection oligonucleotide comprising a plurality of complementarily bound oligonucleotides, a composite detection oligonucleotide comprising a methylated oligonucleotide having a plurality of methylated sites and the like are recited.

The "detection oligonucleotide" in the present invention means an oligonucleotide detectable by the later-described "identification function" and complementarily binding with the test oligonucleotide. The detection oligonucleotide may be any oligonucleotide as far as it has an "identification function" and complementarily binds with the test oligonucleotide, and may be a composite oligonucleotide comprising a plurality of complementarily bound oligonucleotides. When the later-described "detection molecule" is caused to bind as the identification function, the detection oligonucleotide may have the later-described "detection sequence".

The "detection oligonucleotide having a plurality of identification functions" in the present method means that the detection oligonucleotide has a plurality of identification functions. For example, when the detection oligonucleotide is a composite oligonucleotide in which a plurality of oligonucleotides complementarily bind, any oligonucleotide is applicable as far as there are a plurality of identification functions on the composite oligonucleotide, and the positions of the identification functions are not particularly specified. More concretely, it may be the later-described "composite detection oligonucleotide". Also when the detection oligonucleotide is a single-stranded oligonucleotide, any oligonucleotide is applicable as far as there is a plurality of identification functions on the oligonucleotide. For example, when the detection oligonucleotide is a methylated oligonucleotide, methylated DNA having a plurality of identification functions on the detection oligonucleotide may be synthesized and used as a detection oligonucleotide. Since methylated DNA can be detected by using a methylated DNA antibody, the more the number of methylated DNA antibodies binding with the detection oligonucleotide, the higher detection sensitivity is expected. For detecting methylated DNA, there is a method of utilizing the later-described "osmium complex" as well as the method of using a methylated DNA antibody.

When the detection oligonucleotide is a methylated oligonucleotide, it is possible to readily increase the identification functions on the detection oligonucleotide, and to select a detection method suited for the measurement from a variety of detection methods such as a detection method using a methylated DNA antibody and a detection method using an osmium complex.

The "composite detection oligonucleotide" in the present invention is an oligonucleotide that complementarily binds with the test oligonucleotide and are comprising a plurality of complementarily bound oligonucleotides. Also, the composite detection oligonucleotide has the later-described identification function, and has such a structure that a plurality of oligonucleotides are bound by adhesion nucleotide sequences that are possessed by respective oligonucleotides and are mutually complementary nucleotide sequences.

The identification function may be preliminarily bound or indirectly bound to the composite detection oligonucleotide.

The oligonucleotide forming the composite detection oligonucleotide may be entirely or partially methylated. In the present invention, at least partially methylated composite detection oligonucleotide is also called a methylated composite detection oligonucleotide. For forming a composite detection oligonucleotide by making a methylated oligonucleotide complementarily bind, the methylated composite detection oligonucleotide may be such that, for example, only a methylated oligonucleotide is bound, or combination of a methylated oligonucleotide and an unmethylated oligonucleotide are bound.

The "methylated oligonucleotide" means an oligonucleotide in which a base of nucleotide constituting the oligonucleotide is methylated, and in the present invention, it may be the one that is artificially synthesized. It may be prepared by modifying an artificially synthesized oligonucleotide or an oligonucleotide obtained by fragmentating genomic DNA with a methyltransferase. Some methyltransferases are known to methylate position 5 in "CpG" in an oligonucleotide, and concrete examples of such methylase include SssI methylase, and Dmnt1 methylase. Here, since genomic DNA is partially methylated, it is sometimes the case that a region methylated in genome can be obtained as a methylated oligonucleotide by fragmentation of genomic DNA obtained from a cell or the like. Also, the methylated oligonucleotide in which position 5 of cytosine is methylated may be a methylated oligonucleotide that is artificially synthesized by using 5-methylcytosine in place of cytosine. In this case, not only cytosine in "CpG", but also every cytosine (for example, 5'-CA-3', 5'-CT-3', 5'-CC-3' and the like) may be synthesized as 5-methylcytosine.

The "DNA methylation enzyme" means an enzyme that methylates a base in DNA, and various kinds DNA methylation enzymes are isolated from mammalian cells, bacteria and the like. DNA methylation enzymes are classified into several kinds such as adenine methylation enzymes, and cytosine methylation enzymes according to the kind of the base of a substrate. A cytosine methylation enzyme is an enzyme that recognizes a specific sequence in a DNA nucleotide sequence, and methylates cytosine near the sequence, and different cytosine methylation enzymes are known according to the recognized nucleotide sequences.

A number of methylation reactions of DNA catalyzed by a DNA methylation enzyme are found from a primitive immune system called a restriction-modification system. The restriction-modification system is a function that digests foreign DNA (in particular, bacteriophage) with a restriction enzyme after regularly methylating the entire genome functioning in bacteria to protect it from being digested by a restriction enzyme (restriction endonuclease) that recognizes a specific sequence, and is a system for protecting a microbial genome from bacteriophage infection. Enzymes functioning in methylation of genome are known to methylate cytosine or adenine, and often known to methylate nitrogen at position 6 (N6) or carbon at position 5 (C5) of a purine residue. Among these enzymes, known as a cytosine methylation enzyme that methylates C5 of cytosine are SssI (M.SssI) methylase, AluI methylase, HhaI methylase, HpaII methylase, MspI methylase, HaeIII methylase, and so on. These enzymes that methylate position C5 of cytosine recognize different nucleotide sequences, and a cytosine methylation enzyme that recognizes CpG is only SssI.

As a methylation reaction of DNA in human genome, methylation at position 5 (C5) of cytosine in CpG is known as epigenetics (the mechanism generating diversity of gene expression independent of gene sequence), and as such a cytosine methylation enzyme, DNA methyltransferase is known. As a DNA methyltransferase, Dnmtl methyltransferase is known.

In human cells, since position C5 of cytosine in a CpG sequence is methylated, for methylating genome artificially, the same position of the same cytosine in the same sequence (CpG) with methylation in a human cell can be methylated by using SssI.

For making methylated DNA by a cytosine methyltransferase, concretely, for example, the following operation may be conducted. A DNA sample is added with 5 μL of an optimum 10×buffer (NEBuffer2 (available from NEB)), 0.5 μL of S-adenosyl methionine (3.2 mM, available from NEB), and 0.5 μL of cytosine methyltransferase SssI (available from NEB) respectively, and the resultant mixture is added with sterilized ultrapure water to make the liquid amount 50 μL, and then incubated at 37°C for 30 minutes.

In the present invention, when the oligonucleotides constituting the composite detection oligonucleotide bind (link) mutually complementarily in series (also called "serial type"), among the oligonucleotides (including a methylated oligonucleotide) constituting the composite detection oligonucleotide, the oligonucleotide (including a methylated oligonucleotide) having a complementary linkage nucleotide sequence binding with a linkage nucleotide sequence on the test oligonucleotide by complementation is called a first oligonucleotide.

The first oligonucleotide has a first adhesion nucleotide sequence which is an adhesion nucleotide sequence that will not complementarily bind with a nucleotide sequence of the test oligonucleotide, and is able to complementarily bind with a complementary adhesion sequence of a second oligonucleotide which is an oligonucleotide (including a methylated oligonucleotide) capable of complementarily binding with the first oligonucleotide. The second oligonucleotide has a complementary first adhesion nucleotide sequence comprising a nucleotide sequence capable of complementarily binding with the first adhesion nucleotide sequence.

The second oligonucleotide has a second adhesion nucleotide sequence which is an adhesion nucleotide sequence that will not complementarily bind with the test oligonucleotide and a nucleotide sequence part other than the first adhesion sequence in the first oligonucleotide, and is able to complementarily bind with a third oligonucleotide which is an oligonucleotide (including a methylated oligonucleotide) capable of complementarily binding with the second oligonucleotide. The oligonucleotide (including a methylated oligonucleotide) having a complementary second adhesion nucleotide sequence comprising a nucleotide sequence capable of complementarily binding with the second adhesion nucleotide sequence is called a third oligonucleotide.

Similarly, an oligonucleotide (including a methylated oligonucleotide) having a complementary Nth adhesion nucleotide sequence comprising a nucleotide sequence capable of complementarily binding with a Nth adhesion nucleotide sequence is called a (N + 1)th oligonucleotide. The (N + 1)th oligonucleotide has a (N + 1)th adhesion nucleotide sequence which is an adhesion nucleotide sequence that will not complementarily bind with the test oligonucleotide and a nucleotide sequence part of oligonucleotide from the first oligonucleotide to the Nth oligonucleotide other than the Nth adhesion sequence, and is able to complementarily bind with a (N + 2)th oligonucleotide which is an oligonucleotide (including a methylated oligonucleotide) capable of complementarily binding with the (N + 1)th oligonucleotide. This oligonucleotide (including a methylated oligonucleotide) having the complementary (N + 1)th adhesion nucleotide sequence comprising a nucleotide sequence capable of complementarily binding with the (N + 1)th adhesion nucleotide sequence is called a (N + 2)th oligonucleotide.

Similarly, an oligonucleotide (including a methylated oligonucleotide) having a complementary (N - 1)th adhesion nucleotide sequence comprising a nucleotide sequence capable of complementarily binding with a (N - 1)th adhesion nucleotide sequence is called a Nth oligonucleotide. The Nth oligonucleotide has a Nth adhesion nucleotide sequence which is an adhesion nucleotide sequence that will not complementarily bind with the test oligonucleotide and a nucleotide sequence part of oligonucleotide from the first oligonucleotide to the (N - 1)th oligonucleotide other than the (N - 1)th adhesion sequence, and is able to complementarily bind with the (N + 1)th oligonucleotide which is an oligonucleotide (including a methylated oligonucleotide) capable of complementarily binding with the Nth oligonucleotide.

When a (N + 1)th oligonucleotide does not exist, the Nth oligonucleotide is called a terminal oligonucleotide, and the Nth oligonucleotide may not have a Nth adhesion nucleotide sequence.

In other words, one form of the composite detection oligonucleotide in the present invention is such that oligonucleotides from the first oligonucleotide to the terminal oligonucleotide are linked by complementary binding of an adhesion nucleotide sequence and a complementary adhesion nucleotide sequence.

When the composite detection oligonucleotide is formed only of a first oligonucleotide comprising a methylated oligonucleotide having a plurality of methylation sites, the first oligonucleotide may not have an adhesion nucleotide sequence according to the above description.

It is also possible to quantify or detect DNA comprising a target DNA region contained in a specimen by using a detection oligonucleotide comprising a methylated oligonucleotide having only one methylation site without using a composite detection oligonucleotide.

The adhesion nucleotide sequence and the complementary adhesion nucleotide sequence have only to allow complementary binding of oligonucleotide (including a methylated oligonucleotide), and may be located in a terminal end or in the middle of the oligonucleotide (including a methylated oligonucleotide).

Preferably, the Nth adhesion nucleotide sequence will not complementarily bind with a nucleotide sequence other than the complementary Nth adhesion nucleotide sequence, and will not inhibit any complementary binding of nucleotide sequences other than the complementary Nth adhesion nucleotide sequence. Preferably, the Nth adhesion nucleotide sequence will not complementarily bind with nucleotide sequences of oligonucleotides constituting the composite detection oligonucleotide other than the complementary Nth adhesion nucleotide sequence, and nucleotide acids contained in the specimen, and other oligonucleotides including the test oligonucleotide and the later-described specific oligonucleotide.

In the present method, when oligonucleotides constituting a composite detection oligonucleotide can be branched (other than serially) complementarily to each other and bound (linked) (also called "branched"), a plurality of adhesion nucleotide sequences may exist on the Nth oligonucleotide among the oligonucleotides constituting the composite detection oligonucleotide (including methylated oligonucleotide). For example, when there are M adhesion nucleotide sequences in the Nth oligonucleotide, they are called a (N, 1)th adhesion nucleotide sequence, a (N,2)th adhesion nucleotide sequence, a (N,3)th adhesion nucleotide sequence, ... , a (N,(M-1))th adhesion nucleotide sequence, and a (N,M) adhesion nucleotide sequence respectively, and oligonucleotides that bind with these nucleotide sequences by complementation are respectively called a ((N+1), 1)th oligonucleotide, a ((N+1),2)th oligonucleotide, a ((N+1), 3)th oligonucleotide, ... , a ((N+1),(N-1))th oligonucleotide, and a ((N+1), M)th oligonucleotide. In this case, for example, when there is no ((N+2),1)th oligonucleotide, the ((N+1),1)th oligonucleotide is a terminal oligonucleotide, and the ((N+1),1)th adhesion nucleotide sequence may not exist.

Further, similarly, when there are a plurality of adhesion nucleotide sequences on a (N,1)th oligonucleotide, for example, when there are L adhesion nucleotide sequences on the (N,1)th oligonucleotide, they are called a (N,1,1)th adhesion nucleotide sequence, a (N,1,2)th adhesion nucleotide sequence, a (N,1,3)th adhesion nucleotide sequence, ···, a (N,1,(L-1))th adhesion nucleotide sequence, and a (N,1,L)th adhesion nucleotide sequence, respectively, and oligonucleotides that bind with these adhesion nucleotide sequences by complementation are respectively called a ((N+1),1,1)th oligonucleotide, a ((N+1),1,2)th oligonucleotide, a ((N+1),1,3)th oligonucleotide, ···, a ((N+1),1,(L-1))th oligonucleotide, and a ((N+1),1,L)th oligonucleotide. In this case, for example, when there is no ((N+2),1,1)th oligonucleotide, the ((N+1),1,1)th oligonucleotide is a terminal oligonucleotide, and the ((N+1),1,1)th adhesion nucleotide sequence may not exist.

The branched composite oligonucleotide includes the case where plural kinds of first oligonucleotides exist, and the plural kinds of first oligonucleotides bind on the test oligonucleotide. In this case, when there are M first oligonucleotides on the test oligonucleotide, a (1,1)th oligonucleotide, a (1,2)th oligonucleotide, a (1,3)th oligonucleotide, ···, and a (1,M)th oligonucleotide having a (1,1)th adhesion sequence, a (1,2)th adhesion sequence, a (1,3)th adhesion sequence, ···, and a (1,M)th adhesion sequence capable of complementarily binding with a first linkage sequence, a second linkage sequence, ···, and a Nth linkage sequence on the test oligonucleotide can be recited. When there are M second oligonucleotides on the first oligonucleotide, they may be a (2,1)th oligonucleotide, a (2,2)th oligonucleotide, a (2,3)th oligonucleotide, ···, and a (2,M)th oligonucleotide respectively having a (2,1)th adhesion sequence, a (2,2)th adhesion sequence, a (2,3)th adhesion sequence, ···, and a (2,M)th adhesion sequence respectively capable of complementarily binding with a first linkage sequence, a second linkage sequence, ···, and a Mth linkage sequence on the first oligonucleotide can be recited.

Further, when there are M N+1th oligonucleotides on the Nth oligonucleotide, they may be a (N+1,1)th oligonucleotide, a (N+1,2)th oligonucleotide, a (N+1,3)th oligonucleotide, ···, and a (N+1,M)th oligonucleotide respectively having a (N+1,1)th adhesion sequence, a (N+1,2)th adhesion sequence, a (N+1,3)th adhesion sequence, ···, and a (N+1,M)th adhesion sequence, respectively capable of binding with the first linkage sequence, the second linkage sequence, ···, and the Mth linkage sequence on the Nth oligonucleotide.

When there are P N+1th oligonucleotides on the (N,M)th oligonucleotide, they may be a (N+1,M,1)th oligonucleotide, a (N+1,M,2)th oligonucleotide, a (N+1,M,3)th oligonucleotide, ···, and a (N+1,M,P)th oligonucleotide, respectively having a (N+1,M,1)th adhesion sequence, a (N+1,M,2)th adhesion sequence, a (N+1,M,3)th adhesion sequence, ···, and a (N+1,M,P)th adhesion sequence, respectively capable of complementarily binding with the (N+1,M,1)th linkage sequence, the (N+1,M,2)th linkage sequence, ···, and the (N+1,M,P)th linkage sequence on the (N,M)th oligonucleotide.

Further, when there are P N+1th oligonucleotides on the (N,M,···,X)th oligonucleotide, they may be a (N+1,M,···,X,1)th oligonucleotide, a (N+1,M,···,X,2)th oligonucleotide, and a (N+1,M,···,X,3)th oligonucleotide, ···, and a (N+1,M,···,X,P)th oligonucleotide respectively having a (N+1,M,···,X,1)th adhesion sequence, a (N+1,M,···,X,2)th adhesion sequence, and a (N+1,M,···X,3)th adhesion sequence, ···, and a (N+1,M,···,X,P)th adhesion sequence, respectively capable of complementarily binding with the (N+1,M,···, X,1)th linkage sequence, the (N+1,M, ···,X,2)th linkage sequence, ···, and the (N+1,M,···,X,P)th linkage sequence on the (N,M,···,X)th oligonucleotide.

As described above, it is possible to improve the sensitivity by making various combinations, and to prepare a particular combination of an oligonucleotide and a terminal oligonucleotide depending on the sensitivity and accuracy with which the detection is intended to be made.

When a plurality of adhesion nucleotide sequences exists on one oligonucleotide, these adhesion nucleotide sequences may be identical or different from each other. Concretely, for example, nucleotide sequences of said (N,1)th adhesion nucleotide sequence, (N,2)th adhesion nucleotide sequence, and (N,3)th adhesion nucleotide sequence may be identical, or may be nucleotide sequences that are different from each other. The linkage adhesion sequence and the complementary linkage nucleotide sequence, and the adhesion nucleotide sequence and the complementary adhesion nucleotide sequence have only to be nucleotide sequences that are complementarily bindable each other, and concretely, they may have a homology of 90% or higher, and each have usually 5 to 100 bp, preferably 10 to 50 bp. Preferably, the adhesion nucleotide sequence and the complementary adhesion nucleotide sequence are designed so that they will not complementarily bind with genome, and are artificially synthesized DNA. For confirming that the designed adhesion nucleotide sequence, linkage nucleotide sequence or the like fails to complementarily bind with genome in a simple and convenient manner, Blast searching may be executed using a genome database of a public institution such as PubMeD to determine that there is no nucleotide sequence showing a homology of 80% or more.

That is, the "composite detection oligonucleotide" is linked to the test oligonucleotide by complementary binding between the linkage nucleotide sequence and the complementary linkage nucleotide sequence, to form a detection complex.

The detection complex is the one in which a composite detection oligonucleotide is bound to one test oligonucleotide, or the one in which a plurality of composite detection oligonucleotides are bound. In the case where a plurality of composite detection oligonucleotides are bound to one test oligonucleotide, these composite detection oligonucleotides may be the identical composite detection oligonucleotides or different composite detection oligonucleotides. The linkage nucleotide sequence on the test oligonucleotide may be each one of several kinds of linkage nucleotide sequences, or a plurality of one kind of linkage nucleotide sequences.

The "detection complex" in the present invention means the one in which a composite detection oligonucleotide is linked to a test oligonucleotide by complementary binding of the linkage nucleotide sequence of the test oligonucleotide and the complementary linkage nucleotide sequence of the composite detection oligonucleotide. The detection complex has only to have the later-described identification function for quantifying or detecting said DNA comprising a target DNA region in the later-described Third step by detecting the identification function, or is able to bind with a detection molecule having the identification function.

The "identification function" is a function capable of detecting or quantifying a composite detection oligonucleotide. That is, the identification function may be any function capable of identifying a composite detection oligonucleotide, and for example, identification function based on labeling of the composite detection oligonucleotide, and identification function imparted to the detection oligonucleotide by a detection molecule binding with the composite detection oligonucleotide are recited. Concretely, characteristics of fluorescence or coloring of a composite detection oligonucleotide labeled at its 5'-end or 3'-end of the oligonucleotide constituting the composite detection oligonucleotide with europium, gold colloid, latex bead, radioactive isotope, a fluorescent substance (such as FITC), horseradish Peroxidase (HRP), alkaline phosphatase or the like can be recited.

For detection of europium, after adding and mixing Enhancement Solution (available from PerkinElmer, Inc.), and keeping still for about 45 minutes at room temperature, fluorescence (excitation 340nm/fluorescence 612 nm) may be measured by a fluorescent detector. When the composite detection oligonucleotide is a methylated composite oligonucleotide, as a detection molecule, concretely, a methylated DNA antibody, an osmium complex (J. Am. Chem. Soc., 2007; 129:5612-5620) and the like can be recited. Here, concrete examples of the methylated composite oligonucleotide include composite oligonucleotides including 5-methylcytosine, 6-methyladenine and so on. Further, when the composite detection oligonucleotide is labeled with FITC, a FITC antibody can be recited as a detection molecule.

When the detection molecule is a methylated DNA antibody, function as identification function utilized for quantification or detection can be conferred in the following manner. Concretely, labels such as europium label, gold colloid label, latex bead label, radioisotope label, fluorescent substance (e.g., FITC) label, horseradish Peroxidase (HRP) label, alkaline phosphatase label, biotin label and the like are functions using fluorescence, coloring and the like. As a method of imparting an identification function to the antibody functioning as a detection molecule, an identification function may be directly bound to the antibody which is a detection molecule, or a secondary antibody or a tertiary antibody having an identification function may be bound to the antibody which is a detection molecule. Concretely, an antibody labeled with a fluorescent substance, an antibody labeled with horseradish Peroxidase (HRP), an antibody labeled with alkaline phosphatase, an antibody labeled with biotin, and an antibody labeled with europium can be used as a secondary antibody or a tertiary antibody because they are commercially available. Also an antibody to which a substrate detectable by an enzyme cycle method is bound may be used. As a means for quantifying or detecting such function, for example, measurement by a radiation detector, a spectrophotometer or the like, or visual check can be recited. For example, as a case of detecting or quantifying the composite detection oligonucleotide according to its identification function, when a secondary antibody to which europium is imparted as a detectable or quantifiable function is used concretely, Enhancement Solution (available from PerkinElmer Inc.) is added and mixed after allowing the secondary antibody to bind with the detection complex, and left still for about 45 minutes at room temperature. Thereafter, fluorescence (excitation 340 nm/fluorescence 612 nm) may be measured by a fluorescent detector.

When a methylated DNA antibody is allowed to bind with methylated DNA on the composite detection oligonucleotide and detection or quantification is made according to its function, concretely, the following operation may be conducted. After allowing the methylated DNA antibody to bind with the detection complex bound to a support, a secondary antibody against the methylated DNA antibody (for example, Eu-N1-labeled mouse IgG antibody: available from PerkinElmer Inc.) is added, and left still for about 1 hour at room temperature, to thereby prompt binding of the secondary antibody to the detection complex. Thereafter, Enhancement Solution (available from PerkinElmer, Inc.) is added and mixed, and kept still, for example, for about 45 minutes. Then, by measuring fluorescence (excitation 340 nm/fluorescence 612 nm) by a fluorescent detector, detection or quantification is conducted.

When a methylated DNA antibody is used for binding with a support, a methylated DNA antibody having substrate specificity different from that of the methylated DNA antibody used for binding to the support is used as a detection molecule for binding with methylated DNA of the detection oligonucleotide.

For labeling the methylated DNA antibody that binds with methylated DNA on the composite detection oligonucleotide with FITC, an antibody to which FITC is bound may be used as a secondary antibody. In this case, fluorescence of FITC may be measured by a known method to achieve detection or quantification, or detection or quantification may be achieved by using an anti-FITC antibody as a secondary antibody. Further, when FITC is directly bound to the detection oligonucleotide, FITC may be used as an identification function, or labeling function may be imparted by a horseradish Peroxidase (HRP)-labeled FITC antibody, an alkaline phosphatase-labeled FITC antibody, a biotin-labeled FITC antibody, an europium-labeled FITC antibody and the like. Concretely, as the composite detection oligonucleotide, when a FITC-labeled oligonucleotide is used as the composite detection oligonucleotide, after making the detection complex containing the composite detection oligonucleotide bind with a support, an antibody labeled with horseradish Peroxidase (HRP) (for example, HRP-labeled FITC antibody (available from Jackson ImmunoResearch Laboratories Inc.)) is added, and left still for about 1 to 2 hour(s) at room temperature, to prompt binding of the FITC antibody to the detection complex bound to the support. Then after washing and removing the FITC antibody solution, an appropriate substrate (for example, Substrate Reagent Pack #DY999: available from R&D SYSTEMS) is added and mixed. After leaving still at room temperature for about 5 to 60 minutes, a stop solution (2N H2SO4 aqueous solution) is added to stop the reaction of horseradish Peroxidase(HRP), and absorbance at 450 nm may be measured within 30 minutes after stopping of the reaction.

When biotin is not used for immobilizing the test oligonucleotide to the support, a biotinylated detection oligonucleotide can be used for detection or quantification. For detecting or quantifying a biotinylated detection oligonucleotide, for example, HRP-labeled streptavidin is added and mixed to the detection complex immobilized to the support, and a bound body of the biotinylated detection oligonucleotide and the HRP-labeled streptavidin is formed and separated, and then activity of HRP is measured by a known method, so that the biotinylated methylated DNA antibody can be detected or quantified.

As an identification function, a substrate used in a high sensitive detection method such as an enzyme cycle method may be utilized. Concretely, an antibody to which an enzyme used in an enzyme cycle method is immobilized may be immobilized to a detection complex as a detection molecule. The identification function imparted to the detection molecule in the present invention is not limited to the aforementioned method.

The "detection molecule" has only to have a property of detecting or quantifying a composite detection oligonucleotide. The detection molecule may recognize a detection sequence of a composite detection oligonucleotide, or may be bound in advance to a composite detection oligonucleotide. In other words, the detection molecule has only to have a property of specifically binding with a detection oligonucleotide, and having an "identification function" which is a function or characteristic utilized for quantification or detection, or capable of being provided with "identification function". Concretely, when the detection sequence is a methylated oligonucleotide, the detection molecule has only to be able to bind with the methylated oligonucleotide to detect the methylated oligonucleotide, and to specifically bind with the methylated oligonucleotide to exhibit the identification function. (However, when a methylated DNA antibody is used for binding to a support, a methylated DNA antibody having different substrate specificity from that of the methylated DNA antibody used for binding to the support is used as a detection molecule that binds to the methylated DNA of the detection oligonucleotide.) As others, for example, the detection molecule may be a methylated DNA antibody. When the detection sequence is a detection molecule itself, it is not necessary to add a new detection molecule for detecting the composite detection oligonucleotide, and by detecting the detection molecule incorporated into the detection oligonucleotide, it becomes possible to detect the detection oligonucleotide.

The "detection sequence" in the present method means a nucleotide sequence for making a detection molecule bind with a composite detection oligonucleotide.

The detection sequence has only to be a sequence that will not complementarily bind with nucleotide sequences relevant to formation of a detection complex in the present method such as adhesion sequences and complementary adhesion sequences, and may be a synthesized nucleotide sequence, or may have homology with a naturally occurring nucleotide sequence, and it is sufficient that the detection molecule exists in a form capable of binding with the detection complex.

For example, concretely, every composite detection oligonucleotide may have a detection sequence, or only a specific composite detection oligonucleotide contained in the detection complex may have a detection sequence.

The "methylated DNA antibody" in the present invention is an antibody that binds to a methylated base in DNA as its antigen. Concretely, it is a methylcytosine antibody, and an antibody having a property of recognizing and binding to cytosine methylated at position 5 in single-stranded DNA can be recited. Also a commercially available methylated DNA antibody may be applicable as far as it specifically recognizes and specifically binds to DNA in a methylated state as described in the present specification. A methylated DNA antibody can be prepared by an usual immunological technique from a methylated base, methylated DNA or the like as an antigen. Concretely, for preparation of a methylcytosine antibody, it can be obtained by selecting according to specific binding to methyl cytosine in DNA as an index from an antibody that is prepared against DNA containing 5-methylcytidine, 5-methyl cytosine or 5-methyl cytosine as an antigen. Considering the property of the immobilized methylated DNA antibody (one antibody binds to one methylated base (cytosine)), it is preferred to select the region where a number of methylated bases (cytosine) namely CpG exist, as the target DNA region, and improvements in quantification accuracy and detection sensitivity are expected.

As an antibody that is obtainable by immunizing an animal with an antigen, there is a method of using an antibody of IgG fraction (polyclonal antibody), and there is a method of using an antibody producing a single clone (monoclonal antibody) after immunizing with an antigen purified from an animal. In the present invention, since an antibody capable of specifically recognizing methylated DNA or methylcytosine is preferred, use of a monoclonal antibody is preferred.

As a method of preparing a monoclonal antibody, a procedure based on a cell fusion method can be recited. For example, in the cell fusion method, a hybridoma is prepared by allowing cell fusion between a spleen cell (B cell) derived from an immunized mouse and a myeloma cell, and an antibody produced by the hybridoma is selected for preparation of a methyl cytosine antibody (monoclonal antibody). When a monoclonal antibody is prepared by a cell fusion method, it is not necessary to purify an antigen, and for example, a mixture of 5-methyl cytidine, 5-methyl cytosine or DNA or the like containing 5-methyl cytosine may be administered as an antigen to an animal used for immunization. As an administration method, 5-methyl cytidine, 5-methyl cytosine or DNA or the like containing 5-methyl cytosine is directly administered to a mouse for production of an antibody. When an antibody is difficult to be produced, an antigen bound to a support may be used for immunization. Also, by thoroughly mixing an adjuvant solution (prepared, for example, by mixing liquid paraffin and Aracel A, and mixing killed tubercle bacilli as an adjuvant) and an antigen, and immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization. After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse. After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

The "complementarily bind" means that two single-stranded DNA or single-stranded DNA and RNA form double-stranded DNA or a hetero double strand made up of DNA and RNA by base-pairing by a hydrogen bond between bases. For example, a base constituting single-stranded DNA and a base constituting other single-stranded DNA generate base-pairing between purine and pyrimidine, resulting that double-stranded DNA is formed by these single-stranded DNA and more concretely, double-stranded DNA is formed by base-pairing by plural sequential hydrogen bonds between thymine and adenine, and guanine and cytosine. For example, a base constituting single-stranded DNA and a base constituting RNA generate base-pairing between purine and pyrimidine, resulting that a double strand is formed between these single-stranded DNA and RNA, and more concretely, a hetero double strand is formed by base-pairing by plural sequential hydrogen bonds between uracil and adenine, and guanine and cytosine.

The wording "complementarily bind" is also expressed by "complementary binding by base-pairing", "complementary base-pairing" or "bind by complementation". Nucleotide sequences that are capable of complementarily binding are also expressed by "having complementation" or "complementary" to each other. Binding of inosine contained in an artificially prepared oligonucleotide with cytosine, adenine or thymine by hydrogen bonding is also included in complementary binding. The "single-stranded DNA containing a nucleotide sequence that is complementary to a target DNA region" means a nucleotide sequence required for forming a bound body (double-stranded) with single-stranded DNA containing a target DNA region, namely a nucleotide sequence containing a nucleotide sequence that is complementary to a part of the nucleotide sequence of the target DNA region, and is also expressed by "complementary nucleotide sequence".

The "nucleotide sequence showing homology" means a nucleotide sequence having sequence identity. In the present invention, when the percentage of sequence homology is not described, a nucleotide sequence having a sequence identity of 75% or more, preferably 80% or more is meant. Concretely, "nucleotide sequence showing homology with SEQ ID NO: 1" means a nucleotide sequence having a sequence identity of 75% or more and preferably a nucleotide sequence having a sequence identity of 80% or more with the nucleotide sequence of SEQ ID NO: 1 or a partial sequence of SEQ ID NO: 1.

In a second step, the detection complex is immobilized to a support. In the case of immobilizing the detection complex to the support, it may be directly bound to the support by biotinylating 5'-end or 3'-end of the preliminarily obtained test oligonucleotide and conducting the method similar to that described above.

For example, a biotin-labeled test oligonucleotide may be immobilized to an antibody labeled with streptavidin. In such a case, quantification or detection of the test oligonucleotide by the identification function of the composite detection oligonucleotide achieves quantification or detection of the antibody labeled with streptavidin. That is, when the test oligonucleotide is an artificially synthesized oligonucleotide, it may be used for quantification or detection of an object (namely a support) to which the test oligonucleotide is immobilized.

As the support to which the "test oligonucleotide" in the present invention is immobilized, not only detection of DNA or RNA, but also protein such as an antibody is applicable. For example, when the "composite detection oligonucleotide" includes a methylated oligonucleotide, since a plurality of methylated DNA antibodies are able to bind with the "composite detection oligonucleotide", it is possible to detect the support with a detection sensitivity correlated with the number of binding of the methylated DNA antibodies. Generally, in detection of an antibody or the like, since a target molecule is only one (HRP, FITC and the like) for one antibody molecule, improved sensitivity is expected by the present invention. Further, when the methylated oligonucleotide includes 5-methylcytosine, the composite detection oligonucleotide is recognizable by an osmium complex, and improvement in detection sensitivity correlated with the number of 5-methylcytosines contained in the "composite detection oligonucleotide" is expected.

Concretely, for immobilizing a detection complex comprising a composite detection oligonucleotide using up to the third oligonucleotide and the test oligonucleotide to a support by a biotinylated specific oligonucleotide, to genomic DNA aqueous solution (0.1 pmol/10 $\mu$L, in the case of genomic DNA, it is preferred to treat in advance with an appropriate restriction enzyme, to fragmentate the DNA.) containing a test oligonucleotide, each 5 $\mu$L of a first oligonucleotide aqueous solution (0.02 $\mu$M) binding with the test oligonucleotide by complementation, a second oligonucleotide aqueous solution (0.02 $\mu$M) binding with the first oligonucleotide by complementation, a third oligonucleotide aqueous solution (0.02 $\mu$M, in this case, the third oligonucleotide is a terminal oligonucleotide) binding with the second oligonucleotide by complementation, and biotinylated specific oligonucleotide (0.02 $\mu$M) not inhibiting binding of the test oligonucleotide and the composite detection oligonucleotide, and complementarily binding with the test oligonucleotide are added, and further 20 $\mu$L of 100 mM MgCl$_2$, and 10 $\mu$L of an optimum 10 × buffer (330 mM Tris-Acetate pH 7.9, 660 mM KOAc, 100 mM MgOAc2, 5 mM Dithothreitol) are added, and then the resultant mixture was added with sterilized ultrapure water to make the liquid amount 100 $\mu$L, and heated at 95°C for 10 minutes, kept at 70°C for 10 minutes, kept at 50°C for 10 minutes, and then cooled at 37°C for 10 minutes, to obtain a specific detection complex in which the detection complex and the specific oligonucleotide complementarily bind. The specific detection complex formed in this manner may be transferred to an avidin plate, and kept still for 30 minutes at room temperature. Thereafter, the remaining solution is removed and washing is executed. A washing buffer [for example, 0.05% Tween20-containing phosphate buffer (1 mM KH$_2$PO$_4$, 3 mM Na$_2$HPO 7H$_2$O, 154 mM NaCl pH7.4)] is added in a rate of 200 $\mu$L/well, and the solution is removed. This washing operation is repeated several times, so that the detection complex bound to the avidin plate via the specific oligonucleotide is left (selected). From the first oligonucleotide to the terminal oligonucleotide in the above method, at least one or more oligonucleotide(s) should be a methylated oligonucleotide. All oligonucleotides may be methylated oligonucleotides. In the above description, the case up to the third oligonucleotide is shown, a method similar to the above may be conducted up to the Nth oligonucleotide. Although the test oligonucleotide, the biotinylated specific oligonucleotide, and the methylated oligonucleotide (complex) are concurrently added, and the complex is obtained, and then immobilized (selected) by using a biotinylated specific oligonucleotide in the above method, the order is not particularly limited, because it is sufficient that the test methylated oligonucleotide complex is eventually formed and immobilized (selected) in immobilizing (selecting) the test methylated oligonucleotide complex. To be more specific, the biotinylated specific oligonucleotide may be previously immobilized to the avidin plate, and then the test oligonucleotide and the methylated oligonucleotide (complex) may be added, and the test methylated oligonucleotide complex may be obtained and immobilized (selected).

The washing buffer has only to be suited for removal of single-stranded DNA suspended in a solution, and DELFIA buffer (available from PerkinElmer Inc., Tris-HCl pH 7.8 with Tween 80), TE buffer and the like may be used without limited to the aforementioned washing buffer.

As the "support", the material and the shape thereof are not particularly limited as far as the detection complex is bindable thereto. For example, any shape suited for use purpose may be employed, including the shapes of tube, test plate, filter, disc, bead and so on. As the material, those used as supports for a usual immune measuring method, for example, synthetic resins such as polystyrene, polypropylene, polyacrylamide, polymethylmethacrylate, polysulfone, polyacrylonitrile and nylon, or those incorporating a sulfonic group, an amino group or the like reactive functional group into said the synthetic resins can be recited. Also, glass, polysaccharides or derivatives thereof (cellulose, nitrocellulose and the like), silica gel, porous ceramics, metal oxides and the like may be used. The support may be gold colloid (gold nanoparticle) or a latex bead. As the support, it may be a biological molecule such as protein, antibody, lipid or the like biological molecule, or an oligonucleotide.

When "the detection complex is immobilized to the support" in the second step, the detection complex may be immobilized to the support by making the test oligonucleotide bind with the specific oligonucleotide that does not inhibit binding with the composite detection oligonucleotide and complementarily binds with the test oligonucleotide and is able to binding with the support. For making the specific oligonucleotide bind with the support, the specific oligonucleotide has only to have a sequence capable of complementarily binding with a binding function to the support and the test oligonucleotide.

As a method of "forming a detection complex" in the second step of the present invention, concretely, for example, when a detection complex comprising the composite detection oligonucleotide using up to the third oligonucleotide and the test oligonucleotide is obtained, to a genomic DNA aqueous solution (0.1 pmol/10 $\mu$L, in the case of genomic DNA, it is preferred to treat in advance with an appropriate restriction enzyme, to fragmentate the DNA.) containing a test oligonucleotide, each 5 $\mu$L of a first oligonucleotide aqueous solution (0.02 $\mu$M) binding with the test oligonucleotide by complementation, a second oligonucleotide aqueous solution (0.02 $\mu$M) binding with the first oligonucleotide by complementation, a third oligonucleotide aqueous solution (0.02 $\mu$M, in this case, the third oligonucleotide is a terminal oligonucleotide) binding with the second oligonucleotide by complementation are added, and further 20 $\mu$L of 100 mM MgCl$_2$, and 10 $\mu$L of an optimum 10$\times$buffer (330 mM Tris-Acetate pH 7.9, 660 mM KOAc, 100 mM MgOAc2, 5 mM Dithothreitol) are added, and then the resultant mixture was added with sterilized ultrapure water to make the liquid amount 100 $\mu$L, and heated at 95°C for 10 minutes, kept at 70°C for 10 minutes, kept at 50°C for 10 minutes, and then cooled at 37°C for 10 minutes. Of the first oligonucleotide to the terminal oligonucleotide in the above method, at least one or more oligonucleotide(s) should be a methylated oligonucleotide. All oligonucleotides may be methylated oligonucleotides. In the above description, the case up to the third oligonucleotide is shown, a method similar to the above may be conducted up to the Nth oligonucleotide.

The "specific oligonucleotide" in the present invention has only to be an oligonucleotide comprising a nucleotide sequence capable of binding with DNA containing a target DNA region by complementation, and has a function of binding with a support. Concretely, it has a specific adhesion sequence that complementarily binds with DNA comprising a target DNA region and binds with said support. The "specific oligonucleotide" preferably does not inhibit binding of the composite detection oligonucleotide and the test oligonucleotide, and further preferably does not inhibit formation of the composite detection oligonucleotide. Further, it preferably does not complementarily bind with nucleic acid contained in a specimen, and a nucleotide sequence of other oligonucleotide.

The "specific adhesion sequence" is an oligonucleotide comprising a nucleotide sequence complementary to a nucleotide sequence (test oligonucleotide) comprising a target DNA region, and a complementary nucleotide sequence of the nucleotide sequence of the test oligonucleotide with which the specific adhesion sequence is able to pair means having a homology of 75% or higher, preferably 90% or higher with a nucleotide sequence of the specific adhesion sequence. Length of the nucleotide sequence of the specific adhesion sequence is 5 bp to 100 bp, and preferably 10 bp to 50 bp. The specific adhesion sequence has only not to inhibit binding of the detection adhesion sequence and the target DNA region. Also, the "specific adhesion sequence" is preferably a nucleotide sequence that binds with a repetitive sequence in genome, and more preferably a detection adhesion sequence is designed in the same repetitive sequence. Preferably, the detection adhesion sequence and the specific adhesion sequence designed in the same repetitive sequence mutually will not inhibit binding with the test oligonucleotide.

For immobilizing a specific oligonucleotide to a support, concretely a method of immobilizing a biotinylated oligonucleotide obtained by biotinylating 5'-end or 3'-end of the specific oligonucleotide to a support coated with streptavidin (for example, a PCR tube coated with streptavidin, magnetic beads coated with streptavidin, a chromatostrip partially coated with streptavidin and the like) is recited. Also there is a method of letting 5'-end or 3'-end of the specific oligonucleotide covalently bind with a molecule having an active functional group such as an amino group, a thiol group, an aldehyde group or the like, and then letting it covalently bind to a support made of glass, a polysaccharide derivative, silica gel or the synthetic resin or a thermostable plastic whose surface is activated by a silane coupling agent or the like. Covalent binding is achieved, for example, by a spacer formed by serially connecting five triglycerides, a cross linker or the like. Also there is a method of chemically synthesizing from the terminal side of the specific oligonucleotide directly on a support made of glass or silicon.

A third step is a step of quantifying or detecting said DNA comprising a target DNA region by detecting the composite detection oligonucleotide contained in the detection complex formed in the second step according to its identification function.

As a method of "detecting the composite detection oligonucleotide contained in the detection complex formed in the second step according to its identification function" in the third step of the present invention, for example, (1) when a methylated oligonucleotide is used as the test detection oligonucleotide, an antibody (secondary antibody) labeled with europium (hereinafter, also described as "Eu") that binds to the methylated DNA antibody is caused to bind to an avidin plate to which a detection complex is bound, and after adding Enhancement solution (available from PerkinElmer Inc.), fluorescence at excitation 340 nm/fluorescence 612 nm may be measured. FITC label may be used in place of Eu label. To the antibody (secondary antibody) labeled with FITC, a FITC antibody labeled with HRP may further be bound, and detection may be made by enzyme activity of HRP. When detection is made using enzyme activity of HRP, after adding a substrate (R&D systems, Inc., #DY999) and incubating at room temperature, Stop solution (1M $H_2SO_4$:50 $\mu$L/well) may be added, and absorbance at 450 nm (Reference 650 nm) may be measured.

(2) For example, the detection complex bound to the avidin plate obtained in the above concrete method example in each well is added with an appropriate amount of the methylated DNA antibody (for example, 4 $\mu$g/mL solution 100 $\mu$L/well), and left still, for example, for about 3 hours at room temperature, to prompt binding of the methylated DNA antibody and the methylated DNA contained in the composite detection oligonucleotide. Thereafter, the remaining solution is removed and washing is executed. A washing buffer (for example, 0.05% Tween20-containing phosphate buffer (1 mM $KH_2PO_4$, 3 mM Na2HPO 7H2O, 154 mM NaCl pH7.4)) is added, for example, in a rate of 300 $\mu$L/well, and the solution is removed. This washing operation is repeated several times, to leave the detection complex to which the methylated DNA antibody binds on the well. The washing buffer has only to be suited for removal of the aforementioned free methylated DNA antibody, single-stranded DNA suspended in the solution and the like, and DELFIA buffer (available from PerkinElmer Inc., Tris-HCl pH 7.8 with Tween 80), TE buffer and the like may be used without limited to said washing buffer.

(3) For example, each well of an avidin plate was added with 100 $\mu$L of a methylcytosine antibody [available from Aviva Systems Biology, 0.5$\mu$g/mL 0.1% BSA-containing phosphate buffer (1 mM $KH_2PO_4$, 3 mM Na2HPO 7H2O, 154 mM NaCl pH 7.4) solution], and left still for 1 hour at room temperature. Thereafter, the solution is removed by pipetting, and each well is washed three times with 200 $\mu$L of a washing buffer [0.05% Tween20-containing phosphate buffer (1 mM $KH_2PO_4$, 3 mM Na2HPO 7H2O, 154 mM NaCl pH 7.4)]. Further, a secondary antibody against the methylated DNA antibody (for example, Eu-N1-labeled mouse IgG antibody: available from PerkinElmer Inc.) is added, and left still for 1 hour at room temperature, to prompt binding of the secondary antibody to the complex. Thereafter, Enhancement Solution (available from PerkinElmer, Inc.) is added and mixed, and kept still, for example, for about 45 minutes. Thereafter, by measuring fluorescence (excitation 340 nm/fluorescence 612 nm) by a fluorescent detector, the methylated DNA antibody is detected or quantified.

Alternatively, the detection complex bound to the avidin plate is added with a mouse IgG antibody (goat) labeled with FITC prepared into 2 $\mu$g/mL in a rate of 100 $\mu$L/well as a secondary antibody, and left still for 1 hour at room temperature, and then the remaining solution is removed, and a washing buffer [for example, 0.05% Tween20-containing phosphate buffer (1 mM KH2PO4, 3 mM Na2HPO 7H2O, 154 mM NaCl pH7.4)] is added in a rate of 200 $\mu$L/well, and the solution is removed. This washing operation is repeated several times. Further, a tertiary antibody against FITC (for example, HRP-labeled FITC antibody: available from Jackson ImmunoResearch Laboratories) is added to an avidin-coated plate in a rate of 100 $\mu$L/well and incubated at room temperature. A washing buffer [for example, 0.05% Tween20-containing phosphate buffer (1 mM $KH_2PO_4$, 3 mM $Na_2HPO$ $7H_2O$, 154 mM NaCl pH7.4)] is added in a rate of 200 $\mu$L/well, and the solution is removed. This washing operation is repeated several times. A substrate (R&D Systems, Inc., #DY999) is added in a rate of 100 $\mu$L/well, and stirred for about 10 seconds. After incubating at room temperature, Stop solution (1M $H_2SO_4$: 50 $\mu$L/well) is added and stirred for about 10 seconds. In 30 minutes, absorbance at 450 nm (Reference 650 nm) is measured (light shielding is preferred).

In the present invention, as a method of quantifying or detecting RNA comprising a target RNA region, first, RNA comprising a target RNA region may be obtained from a biological specimen.

For example, concretely, for obtaining RNA from a biological specimen, RNA may be extracted, for example, by using a commercially available RNA extraction kit.

Next, a bound body with the test detection oligonucleotide capable of complementarily binding with RNA comprising a target RNA region is immobilized to a support, and the detection may be made according to the identification function of the test detection oligonucleotide. For immobilizing the detection complex comprising the RNA comprising a target RNA region and the test detection oligonucleotide to the support, a specific oligonucleotide that is an oligonucleotide capable of complementarily binding with the RNA comprising a target RNA region constituting the detection complex, and having a function of binding to the support may be added in forming the detection complex, to form a complex comprising the RNA comprising a target RNA region, the test detection oligonucleotide and the specific oligonucleotide, whereby immobilization to the support may be achieved.

For "forming a complex comprising the RNA comprising a target RNA region, the test detection oligonucleotide and the specific oligonucleotide", for example, RNA extracted from the biological specimen may be immobilized to the support by forming a complex of the detection complex in which the "linear type" composite detection oligonucleotide comprising the first oligonucleotide to the third oligonucleotide, and the RNA comprising a target RNA region complementary bind, and a biotinylated specific oligonucleotide. Concretely, an aqueous solution containing the RNA (0.1 pmol/ 10 $\mu$L, prepared with an RNAse free aqueous solution. Concretely, an aqueous solution is prepared using water treated at 120 atmospheric pressures for 20 minutes and so on.) is added with each 5 $\mu$L of an aqueous solution of a first oligonucleotide complementarily binding with the RNA by complementation (0.02 $\mu$M), an aqueous solution of a second oligonucleotide binding with the first oligonucleotide by complementation (0.02 $\mu$M), an aqueous solution of a third oligonucleotide binding with the second oligonucleotide by complementation (0.02 $\mu$M, in this case, the third oligonucleotide is a terminal oligonucleotide), and a biotinylated specific oligonucleotide (0.02 $\mu$M) that will not inhibit binding of the RNA and the composite detection oligonucleotide, and complementarily binds with the RNA, to prepare a mixture (containing 50% formamide, 5 $\times$ SSC (150 mM NaCL, 15 mM trisodium citrate), 50 mM sodium phosphate (pH 7.6), 5 $\times$ Denhardt's solution, 10% dextran sulfate, and 20 $\mu$g/mL denatured sheared salmon sperm DNA), and the liquid amount of the mixture is adjusted to 100 $\mu$L, and heated at 95°C for 10 minutes, kept at 70°C for 10 minutes, kept at 50°C for 10 minutes, and then cooled at 37°C for 10 minutes, to obtain a specific detection complex in which the detection complex and the specific oligonucleotide complementarily bind. The mixture is a general hybridization solution, and is a solution used in a well-known hybridization method as described in Molecular Cloning, A Laboratory Manual, 2nd Ed., Cold Spring Harbor Laboratory (1989).

For making "the complex comprising the RNA comprising a target RNA region, the test detection oligonucleotide and the specific oligonucleotide" be "immobilized to the support", concretely, a complex formed by allowing a biotinylated specific oligonucleotide complementarily bind to the detection complex in which the "linear" composite detection oligonucleotide formed of the first oligonucleotide to the third oligonucleotide and the RNA comprising a target RNA region complementarily bind is transferred to an avidin plate, and left still for 30 minutes at room temperature. Thereafter, the remaining solution is removed and washing is executed. A washing buffer [for example, 0.05% Tween20-containing phosphate buffer (1 mM $KH_2PO_4$, 3 mM $Na_2HPO$ $7H_2O$, 154 mM NaCl pH7.4)] is added in a rate of 200 $\mu$L/well, and the solution is removed. This washing operation is repeated several times, to leave (select) the detection complex bound to the avidin plate via the specific oligonucleotide. Of the first oligonucleotide to the terminal oligonucleotide in the above method, at least one or more oligonucleotide(s) should be a methylated oligonucleotide. All oligonucleotides may be methylated oligonucleotides. In the above description, while the case up to the third oligonucleotide is shown, a method similar to the above may be conducted up to the Nth oligonucleotide. Although the test oligonucleotide, the biotinylated specific oligonucleotide, and the methylated oligonucleotide (complex) are concurrently added, and the complex is obtained, and then immobilized (selected) by using a biotinylated specific oligonucleotide in the above method, the order is not particularly limited because it is sufficient that the test methylated oligonucleotide complex is eventually formed and immobilized (selected) in immobilizing (selecting) the test methylated oligonucleotide complex. To be more specific, the biotinylated specific oligonucleotide may be previously immobilized to the avidin plate, and then the test oligonucleotide and the methylated oligonucleotide (complex) may be added, to obtain and immobilize (select) the test methylated oligonucleotide complex.

As a method of quantifying or detecting RNA comprising a target RNA region in the present invention, for quantifying or detecting "a complex formed by letting the biotinylated specific oligonucleotide complementarily bind with the detection complex formed by complementary binding of the "linear" composite detection oligonucleotide comprising the first oligonucleotide to the third oligonucleotide and the RNA comprising a target RNA region", identification function of the test detection oligonucleotide may be used. Concretely, each well of the avidin plate is added with 100 $\mu$L of a methylcytosine antibody [available from Aviva Systems Biology, 0.5 $\mu$g/mL 0.1% BSA-containing phosphate buffer (1 mM $KH_2PO_4$, 3 mM Na2HPO $7H_2O$, 154 mM NaCl pH 7.4) solution], and left still for 1 hour at room temperature. Thereafter, the solution is removed by pipetting, and each well is washed three times with 200 $\mu$L of a washing buffer [0.05% Tween20-containing phosphate buffer (1 mM $KH_2PO_4$, 3 mM Na2HPO $7H_2O$, 154 mM NaCl pH 7.4)]. Further, a secondary antibody against the methylated DNA antibody (for example, Eu-N1-labeled mouse IgG antibody: available from PerkinElmer) is added, and kept still at room temperature for about 1 hour, to prompt binding of the complex to the secondary antibody. Thereafter, Enhancement Solution (available from PerkinElmer, Inc.) is added and mixed, and kept still, for example, for about 45 minutes. Thereafter, by detecting or quantifying the methylated DNA antibody by measuring fluorescence (excitation 340 nm/fluorescence 612 nm) with a fluorescent detector, a measurement correlated with a target region of the RNA contained in the biological specimen can be obtained.

The present invention may be used in the following situations.

In various diseases, by quantifying or detecting RNA itself showing correlation with the degree of such a disease, DNA prepared from the RNA as a template, DNA showing correlation with the degree of such a disease and the like, the degree of such a disease can be estimated. For example, in cancer or the like, it may be used as a screening test in a regular health examination by quantification of free DNA in blood. In infection or the like, by detecting or quantifying DNA or RNA of a bacterium or virus which is a cause of the disease, or DNA prepared from the RNA as a template by a reverse transcriptase, the causative bacterium or the causative virus would be identified. Also the present invention enables detection of DNA without conducting a complicated method such as PCR for the DNA that has been conventionally detected after amplifying the DNA by executing PCR or the like because of its small amount, or for RNA that has been detected after synthesizing DNA by a reverse transcriptase. Also it becomes possible to quantify or detect RNA without synthesizing DNA by a reverse transcriptase.

As a method of detecting or quantifying minor substances contained in a biological sample such as blood or urine, immunological measuring methods are generally used. Among such immunological measuring methods, a so-called immune chromatography using chromatography is widely used in various situations including, for example, clinical examinations in hospitals, assays in laboratories and the like because of its simple operation and short time required for assay. In recent years, a so-called hybrid chromatography has been utilized wherein labeled DNA (gene) is developed on a chromatostrip, and target DNA (gene) is detected by hybridization using a probe capable of capturing the target DNA (gene). Also this method is now coming to be widely used in situations including, for example, clinical examinations in hospitals, assays in laboratories and the like because of its simple operation and short required time for assay. The present measurement method conceptually enables a combined method of the immune chromatography and the hybrid chromatography. In the present method, since the order of formation of a complex and obtainment of a complex is not particularly limited, various methods are possible. Concretely, such methods may be executed in the following manner.

For example, to a sample directly after end of Second step, a biotinylated specific oligonucleotide and a detection oligonucleotide having an identification function are added, and the methylated single-stranded DNA containing a target DNA region, the detection oligonucleotide having an identification function and the biotinylated specific oligonucleotide are allowed to bind each other, to thereby form a detection complex in which a bound body of the single-stranded DNA containing a target DNA region, the detection oligonucleotide having an identification function, and the biotinylated specific oligonucleotide is bound to the support. As the obtained sample is dropped (applied) into an applying part of a chromatostrip, said complex migrates in a development part by a capillary phenomenon, and is trapped in the part preliminarily coated with streptavidin. Then by detecting or quantifying the detection oligonucleotide contained in the obtained complex according to its identification function, DNA comprising a target DNA region can be detected or quantified.

It is also possible to make a plurality of detection sites (using detection oligonucleotides respectively capable of complementarily binding with different target DNA regions) exist in the target DNA region, and detect or quantify these target DNA regions sequentially. Also, detection sensitivity can be dramatically improved by using a detection oligonucleotide capable of complementarily binding with a plurality of target DNA regions in such a manner that a repetitive sequence in genome, a duplicate gene or a plurality of different genes are concurrently detected so that a complex will be formed with a plurality of target DNA regions. Further, by designing a number of detection oligonucleotides in a single target region, and using these on the support side or on the detection side, the detection sensitivity can be dramatically improved.

As a method of conducting a process of forming a complex of a detection oligonucleotide, a biotinylated specific oligonucleotide, and a target DNA region or a target RNA region and making it bind with a support, any method using a immune antibody method may be used without limited to the aforementioned method. For example, in the ELISA method, a process of forming a complex and making it bind with a support can be executed in the described order because the principle similar to that of the chromatostrip method is used.

The methylated oligonucleotide or the like in the present invention is useful as a reagent of a detection kit. The scope of the present method includes use in the form of a detection kit as described above using the substantial principle of the present method.

Of nucleotide sequences published on a database, a nucleotide sequence peculiar to a microorganism can be searched. For example, a nucleotide sequence on a published database such as PubMed may be obtained through regular procedure, and the obtained nucleotide sequence can be examined whether it is a peculiar nucleotide sequence by Blast search through regular procedure. The peculiar nucleotide sequence means that the nucleotide sequence to be detected does not have a nucleotide sequence showing homology with a nucleotide sequence originating from an organism other than the microorganism to be detected.

In particular, when the specimen is a human biopsy sample, it is important to design a specific oligonucleotide that will not complementarily bind with human genes. Similarly, when the specimen is food, it is important to design an adhesion nucleotide sequence and a specific oligonucleotide that will not complementarily bind with a nucleotide sequence derived from an organism other than the object to be detected contained in the food.

When one wants to examine a repetitive sequence in a certain region, it is difficult to carry out a search on a general sequence retrieving database such as PubMed, and in general, Repbase (http://www.girinst.org/repbase/), RepeatMasker (http://www.repeatmasker.org/) and the like database may be used. It is possible to improve the detection sensitivity if a specific adhesion sequence and a detection adhesion sequence of the present method can be set. Measuring these repetitive sequences, for example, enables a free DNA amount in blood to be treated as a surrogate marker, which can be used for identification of an organism species when a repetitive sequence specific to an organism species is focused.

The "labeling method of a specimen using a composite detection oligonucleotide" in the present method means a method of labeling a test oligonucleotide by allowing binding of a composite detection oligonucleotide in which a plurality of oligonucleotides complementarily bind. For example, when a method that detects methylated DNA as identification function of the composite detection oligonucleotide is used, since an unlimited number of methylated DNA can be designed on the composite detection oligonucleotide in principle, increase in detection sensitivity correlated with the number of methylated DNA designed on the composite detection oligonucleotide can be expected. The present method also includes a method of increasing the detection sensitivity by using a composite detection oligonucleotide as described above.

The detection oligonucleotide may comprise one methylated oligonucleotide, and in this case, improvement in detection sensitivity correlated with the number of methylated DNA designed on the detection oligonucleotide can be expected. That is, in the present method, by using the methylated oligonucleotide as a detection oligonucleotide, detection sensitivity correlated with the number of methylated DNA designed on the detection oligonucleotide can be expected as is the case with the improvement in detection sensitivity by the composite detection oligonucleotide as described above.

In the present method, the "labeling method of a specimen using a composite detection oligonucleotide and a reagent capable of labeling the composite detection oligonucleotide" includes any method combining a labeling method by a composite detection oligonucleotide and identification function of the composite detection oligonucleotide. For example, in the case where identification function of the composite detection oligonucleotide utilizes methylated DNA, when the composite detection oligonucleotide is detected by a methylated DNA antibody, a labeling method using the composite detection oligonucleotide and the methylated DNA antibody is recited. For example, when the composite detection oligonucleotide is labeled by complementary binding of the fluorescently labeled oligonucleotide, a labeling method using the composite detection oligonucleotide and the fluorescently labeled oligonucleotide can be recited.

EXAMPLES

In the following, the present invention will be described in detail by way of examples, however, the present invention is not limited to these examples.

Example 1

An oligonucleotide comprising the nucleotide sequence of SEQ ID NO: 1 was synthesized as a test oligonucleotide, and the following TE buffer solutions were prepared.

Solution A: test oligonucleotide 0 pmol/10 $\mu$L TE buffer solution (negative control solution)
Solution B: test oligonucleotide 0.001 pmoL/10 $\mu$L TE buffer solution
Solution C: test oligonucleotide 0.01 pmoL/10 $\mu$L TE buffer solution

<Test oligonucleotide>

5'-AGTGACACCATCGAGAATGTCAGATCCGGATCAGAGCGCCATCTAGATGGACATGTCACTGT CTGACTACAACATCCAGA -3' (SEQ ID NO: 1)

As a specific oligonucleotide used for obtaining a test oligonucleotide, a 5'-end biotinylated oligonucleotide comprising the nucleotide sequence of SEQ ID NO: 2 was synthesized, and a 0.1 pmoL/5 $\mu$L TE buffer solution was prepared.

<5'-end biotinylated oligonucleotide>

5'- Biotin- TCTGGATGTTGTAGTCAGACAG -3' (SEQ ID NO: 2)
For detecting a test oligonucleotide, as a fluorescence-modified oligonucleotide used for general DNA detection (for Control 1 treatment group), a 5'-end FITC-labeled oligonucleotide comprising the nucleotide sequence of SEQ ID NO: 3 to which a fluorescein antibody is able to bind at one site was synthesized, and a 0.1 pmoL/5 $\mu$L TE buffer solution was prepared.

<5'-end FITC-labeled oligonucleotide>

5'-FITC-TGACATTCTCGATGGTGTCACT-3' (SEQ ID NO: 3)
Also for detecting a test oligonucleotide, as a fluorescence-modified oligonucleotide used for general DNA detection (for Control 2 treatment group), a 3'-end FLC-labeled oligonucleotide comprising the nucleotide sequence of SEQ ID NO: 4 to which a fluorescein antibody is able to bind at one site was synthesized, and a 0.1 pmoL/5 $\mu$L TE buffer solution was prepared.

<3'-end FLC-labeled oligonucleotide>

5'-TGACATTCTCGATGGTGTCACTCACACACACACACACACACACAGACAACGCCTCGTTCT CGG-FLC -3' (SEQ ID NO: 4)
As a methylated oligonucleotide (first oligonucleotide, for X treatment group) that binds with a test oligonucleotide by complementation for detecting the test oligonucleotide, a methylated oligonucleotide M1 comprising the nucleotide sequence of SEQ ID NO: 5 to which a methylcytosine antibody is able to bind at one site was synthesized, and a 0.1 pmoL/5 $\mu$L TE buffer solution was prepared.

<Methylated oligonucleotide M1> N represents methylated cytosine.

5'-TGACATTCTCGATGGTGTCACTCACACACACACACACACACANAGACAACGCCTCGTTCT CGG -3' (SEQ ID NO: 5)
As a methylated oligonucleotide (first oligonucleotide, for Y treatment group) that binds with a test oligonucleotide by complementation for detecting the test oligonucleotide, a methylated oligonucleotide M12A comprising the nucleotide sequence of SEQ ID NO: 6 to which a methylcytosine antibody is able to bind at 12 sites was synthesized, and a 0.1 pmoL/5 $\mu$L TE buffer solution was prepared.

<Methylated oligonucleotide M12A> N represents methylated cytosine.

5'-TGACATTCTCGATGGTGTCACTNANANANANANANANANANANANANAGACAACGCCTCGTTCT CGG -3' (SEQ ID NO: 6)

For the solutions of the test oligonucleotide, the specific oligonucleotide and the methylated (or fluorescence-modified) oligonucleotide obtained above, the following four treatments (Control 1 treatment group, Control 2 treatment group, X treatment group, and Y treatment group) were conducted.

<Control 1 treatment group>

In a PCR tube, 10 $\mu$L of the test oligonucleotide solution prepared in the above, 5 $\mu$L of said specific oligonucleotide solution, 5 $\mu$L of said 5'-end FITC-labeled oligonucleotide solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the 5'-end FITC-labeled oligonucleotide (that is, for forming a complex of the test oligonucleotide, the specific oligonucleotide and the 5'-end FITC-labeled oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

<Control 2 treatment group>

In a PCR tube, 10 $\mu$L of the test oligonucleotide solution prepared in the above, 5 $\mu$L of said specific oligonucleotide solution, 5 $\mu$L of said 3'-end FLC-labeled oligonucleotide solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the 3'-end FLC-labeled oligonucleotide (that is, for forming a complex of the test oligonucleotide, the specific oligonucleotide and the 3'-end FLC-labeled oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

<X treatment group>

In a PCR tube, 10 $\mu$L of the test oligonucleotide solution prepared in the above, 5 $\mu$L of said specific oligonucleotide solution, 5 $\mu$L of said methylated oligonucleotide M1 solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the methylated oligonucleotide M1 (that is, for forming a complex of the test oligonucleotide, the specific oligonucleotide and the methylated oligonucleotide M1), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

<Y treatment group>

In a PCR tube, 10 $\mu$L of the test oligonucleotide solution prepared in the above, 5 $\mu$L of said specific oligonucleotide solution, 5 $\mu$L of said methylated oligonucleotide M12A solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the methylated oligonucleotide M12A (that is, for forming a complex of the test oligonucleotide, the specific oligonucleotide and the methylated oligonucleotide M12A), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

The entire obtained mixture was transferred to a 8-well strip coated with streptavidin, and left still for about 30 minutes at room temperature, to immobilize the complex of the test oligonucleotide, the specific oligonucleotide and the methylated (or fluorescence-modified) oligonucleotide to the 8-well strip via biotin-streptavidin bond. Thereafter, the solution was removed by decantation, and each well was washed three times with 200 $\mu$L of a washing buffer [0.05% Tween20-containing phosphate buffer (1 mM $KH_2PO_4$, 3 mM $Na_2HPO_4$ $7H_2O$, 154 mM NaCl pH7.4)].

As to the subsequent operation, the following different treatments were conducted for Control 1 treatment group and Control 2 treatment group, and for X treatment group and Y treatment group.

For Control 1 treatment group and Control 2 treatment group (the cases using 5'-end FITC-labeled oligonucleotide and 3'-end FLC-labeled oligonucleotide), the following treatment was conducted.

Each well was added with 100 $\mu$L of an antibody solution [Peroxidase-conjugated IgG Fraction Monoclonal Mouse Anti-Fluorescein: available from Jackson ImmunoResearch Laboratories Inc., 0.1 $\mu$g/mL 0.1% BSA-containing phosphate buffer (1 mM $KH_2PO_4$, 3 mM $Na_2HPO_4$ $7H_2O$, 154 mM NaCl pH 7.4) solution] and left still for 1 hour at room temperature. Then each well was washed three times with 200 $\mu$L of a washing buffer [0.05% Tween20-containing phosphate buffer (1 mM $KH_2PO_4$, 3 mM $Na_2HPO_4$ $7H_2O$, 154 mM NaCl pH 7.4)].

Each well was added and mixed with 100 $\mu$L of a substrate (available from R&D systems Inc., #DY999), to initiate the reaction.

After leaving still for about 5 minutes at room temperature, each well was added with 50 $\mu$L of a stop solution (1N $H_2SO_4$ aqueous solution), to stop the reaction. Within 30 minutes after stopping of the reaction, absorbance at 450 nm was measured.

For X treatment group and Y treatment group (the cases using the methylated oligonucleotide M1 and the methylated oligonucleotide M12A), the following treatment was conducted.

Each well was added with 100 $\mu$L of a primary antibody solution [methylcytosine antibody: available from AVIVA, 1 $\mu$g/mL 0.1% BSA-containing phosphate buffer (1 mM $KH_2PO_4$, 3 mM $Na_2HPO_4$ $7H_2O$, 154 mM NaCl pH 7.4) solution], and left still for 1 hour at room temperature. Thereafter, each well was washed three times with 200 $\mu$L of a washing buffer [0.05% Tween20-containing phosphate buffer (1 mM $KH_2PO_4$, 3 mM $Na_2HPO_4$ $7H_2O$, 154 mM NaCl pH7.4)].

Then each well was added with 100 $\mu$L of a secondary antibody solution [mouse IgG antibody FITC (derived from goat): available from MBL, 2 $\mu$g/mL 0.1% BSA-containing phosphate buffer (1 mM $KH_2PO_4$, 3 mM $Na_2HPO_4$ $7H_2O$, 154 mM NaCl pH 7.4) solution], and left still for 1 hour at room temperature. Thereafter, each well was washed three times with 200 $\mu$L of a washing buffer [0.05% Tween20-containing phosphate buffer (1 mM $KH_2PO_4$, 3 mM $Na_2HPO_4$ $7H_2O$, 154 mM NaCl pH 7.4)].

Each well was added with 100 $\mu$L of a tertiary antibody solution [Peroxidase-conjugated IgG Fraction Monoclonal Mouse Anti-Fluorescein: available from Jackson ImmunoResearch Laboratories Inc., 0.1 $\mu$g/mL 0.1% BSA-containing phosphate buffer (1 mM $KH_2PO_4$, 3 mM $Na_2HPO_4$ $7H_2O$, 154 mM NaCl pH 7.4) solution], and left still for 1 hour at room temperature. Thereafter, each well was washed three times with 200 $\mu$L of a washing buffer [0.05% Tween20-containing phosphate buffer (1 mM $KH_2PO_4$, 3 mM $Na_2HPO_4$ $7H_2O$, 154 mM NaCl pH 7.4)].

Each well was added and mixed with 100 $\mu$L of a substrate (available from R&D systems Inc., #DY999), to initiate the reaction.

After leaving still for about 5 minutes at room temperature, each well was added with 50 $\mu$L of a stop solution (1N $H_2SO_4$ aqueous solution), to stop the reaction. Within 30 minutes after stopping of the reaction, absorbance at 450 nm was measured.

For analysis of data, the following method was used. For making the values of negative control solutions constant among every group, every measured value is divided by a measured value of a negative control solution of each group, and multiplied by a minimum value of negative control solution of all groups. Then a minimum value of negative control solution of all groups is subtracted from each resultant value, and the result is used as a corrected value.

```
[Corrected value] = [Measured value] x [Minimum value]/[Value

of negative control solution of each group] - [Minimum value]
```

The result is shown in Fig. 1. In the present Example, detection sensitivity was generally equal in Control 1 treatment group, Control 2 treatment group, and X group. Therefore, it was proved that the detection sensitivity is not largely different from that of the conventional detection method when the site to which the methylcytosine antibody is bindable is one (there is one methylated cytosine). In Y treatment group using a methylated oligonucleotide having many sites (12 sites) to which a methylcytosine antibody is bindable, it was revealed that detection sensitivity is higher than in Control 1 treatment group, Control 2 treatment group, and X group. That is, it was proved that detection sensitivity improves by using an oligonucleotide having many sites (there are many methylated cytosines) to which a methylcytosine antibody is bindable.

Example 2

An oligonucleotide comprising the nucleotide sequence of SEQ ID NO: 1 was synthesized as a test oligonucleotide, and the following TE buffer solutions were prepared.

Solution A: test oligonucleotide 0 pmol/10 $\mu$L TE buffer solution (negative control solution)
Solution B: test oligonucleotide 0.0001 pmoL/10 $\mu$L TE buffer solution
Solution C: test oligonucleotide 0.001 pmoL/10 $\mu$L TE buffer solution
Solution D: test oligonucleotide 0.01 pmoL/10 $\mu$L TE buffer solution

<Test oligonucleotide>

5'-AGTGACACCATCGAGAATGTCAGATCCGGATCAGAGCGCCATCTAGATGGACATGTCACTGT CTGACTACAACATCCAGA -3' (SEQ ID NO: 1)
As a specific oligonucleotide used for obtaining a test oligonucleotide, a 5'-end biotinylated oligonucleotide comprising the nucleotide sequence of SEQ ID NO: 2 was synthesized, and a 0.1 pmoL/5 $\mu$L TE buffer solution was prepared.

<5'-end biotinylated oligonucleotide>

5'- Biotin- TCTGGATGTTGTAGTCAGACAG -3' (SEQ ID NO: 2)
As a methylated oligonucleotide (first oligonucleotide, for X treatment group) that binds with a test oligonucleotide by complementation for detecting the test oligonucleotide, a methylated oligonucleotide M1 comprising the nucleotide sequence of SEQ ID NO: 5 to which a methylcytosine antibody is able to bind at one site was synthesized, and a 0.1 pmoL/5 $\mu$L TE buffer solution was prepared.

<Methylated oligonucleotide M1> N represents methylated cytosine.

5'-TGACATTCTCGATGGTGTCACTCACACACACACACACACACACANAGACAACGCCTCGTTCT CGG -3' (SEQ ID NO: 5)
As a methylated oligonucleotide (first oligonucleotide, for Y treatment group) that binds with a test oligonucleotide by complementation for detecting the test oligonucleotide, a methylated oligonucleotide M12A comprising the nucleotide sequence of SEQ ID NO: 6 to which a methylcytosine antibody is able to bind at 12 sites was synthesized, and a 0.1 pmoL/5 $\mu$L TE buffer solution was prepared.

<Methylated oligonucleotide M12A> N represents methylated cytosine.

5'-TGACATTCTCGATGGTGTCACTNANANANANANANANANANANANANAGACAACGCCTCGTTCT CGG -3' (SEQ ID NO: 6)
For the solutions of the test oligonucleotide, the specific oligonucleotide and the methylated oligonucleotide obtained above, the following two treatments (X treatment group, Y treatment group) were conducted.

<X treatment group>

In a PCR tube, 10 $\mu$L of the test oligonucleotide solution prepared in the above, 5 $\mu$L of said specific oligonucleotide solution, 5 $\mu$L of said methylated oligonucleotide M1 solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the methylated oligonucleotide M1 (that is, for forming a complex of the test oligonucleotide, the specific oligonucleotide and the methylated oligonucleotide M1), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

<Y treatment group>

In a PCR tube, 10 $\mu$L of the test oligonucleotide solution prepared in the above, 5 $\mu$L of said specific oligonucleotide solution, 5 $\mu$L of said methylated oligonucleotide M12A solution, 10 $\mu$L of a buffer (330 mM Tris-Acetate pH 7.9, 660 mM KOAc, 100 mM MgOAc$_2$, 5 mM Dithiothreitol), and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the methylated oligonucleotide M12A (that is, for forming a complex of the test oligonucleotide, the specific oligonucleotide and the methylated oligonucleotide M12A), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

The entire obtained mixture was transferred to a 8-well strip coated with streptavidin, and left still for about 30 minutes at room temperature, to immobilize the complex of the test oligonucleotide, the specific oligonucleotide and the methylated oligonucleotide to the 8-well strip via biotin-streptavidin bond. Thereafter, the solution was removed by decantation, and each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH7.4)].

Each well was added with 100 $\mu$L of a primary antibody solution [methylcytosine antibody: available from AVIVA, 1$\mu$ g/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. Thereafter, each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Then each well was added with 100 $\mu$L of a secondary antibody solution [mouse IgG antibody Eu-N1: 0.25 $\mu$g/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. After leaving still, each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Each well was added and mixed with 200 $\mu$L of Enhancement Solution, and shaken for 5 minutes at room temperature using a plate shaker. Thereafter, fluorescence was measured at excitation 340 nm/fluorescence 612 nm.

For analysis of data, the following method was used. For making the values of negative control solutions constant among every group, every measured value is divided by a measured value of a negative control solution of each group, and multiplied by a minimum value of negative control solution of all groups. Then a minimum value of negative control solution of all groups is subtracted from each resultant value, and the result is used as a corrected value.

```
[Corrected value] = [Measured value] x [Minimum value]/[Value

of negative control solution of each group] - [Minimum value]
```

The result is shown in Fig. 2. In the present example, it was revealed that detection sensitivity is higher in Y group using a methylated oligonucleotide having many sites (12 sites) than in X group. That is, it was proved that detection sensitivity improves by using an oligonucleotide having many sites (there are many methylated cytosines) to which a methylcytosine antibody is bindable.

Example 3

An oligonucleotide comprising the nucleotide sequence of SEQ ID NO: 1 was synthesized as a test oligonucleotide, and the following TE buffer solutions were prepared.

Solution A: test oligonucleotide 0 pmol/10 $\mu$L TE buffer solution (negative control solution)
Solution B: test oligonucleotide 0.003 pmoL/10 $\mu$L TE buffer solution
Solution C: test oligonucleotide 0.01 pmoL/10 $\mu$L TE buffer solution
Solution D: test oligonucleotide 0.03 pmoL/10 $\mu$L TE buffer solution

<Test oligonucleotide>

5'-AGTGACACCATCGAGAATGTCAGATCCGGATCAGAGCGCCATCTAGATGGACATGTCACTGT CTGACTACAACATCCAGA -3' (SEQ ID NO: 1)

As a specific oligonucleotide used for obtaining a test oligonucleotide, a 5'-end biotinylated oligonucleotide comprising the nucleotide sequence of SEQ ID NO: 2 was synthesized, and a 0.1 pmoL/5 $\mu$L TE buffer solution was prepared.

<5'-end biotinylated oligonucleotide>

5'- Biotin- TCTGGATGTTGTAGTCAGACAG -3' (SEQ ID NO: 2)

As an oligonucleotide that binds with a test oligonucleotide by complementation for detecting the test oligonucleotide, a methylated oligonucleotide (first oligonucleotide) comprising the nucleotide sequence of SEQ ID NO: 7 to which a methylcytosine antibody is able to bind at three sites was synthesized, and a 0.1 pmoL/5 $\mu$L TE buffer solution was prepared. This first oligonucleotide has a first adhesion nucleotide sequence which is an adhesion nucleotide sequence at 3'-end.

<First oligonucleotide> N represents methylated cytosine.

5'- TGACATTCTCGATGGTGTCACTANACANACANATGCGCACCGTGCGCGAGC -3' (SEQ ID NO: 7)

As an oligonucleotide having a complementary first adhesion nucleotide sequence (binding with a first oligonucleotide by complementation) comprising a nucleotide sequence capable of complementarily binding with the first adhesion nucleotide sequence for detecting the test oligonucleotide, an unmethylated oligonucleotide comprising the nucleotide sequence of SEQ ID NO: 8 (second oligonucleotide) was synthesized, and a 0.1 pmoL/5 $\mu$L TE buffer solution was prepared. This second oligonucleotides has a second adhesion nucleotide sequence which is an adhesion nucleotide sequence at 5'-end.

<Second oligonucleotide>

5'- ATAGTCTCGTGGTGCGCCGTACACACACACAGCTCGCGCACGGTGCGCA -3' (SEQ ID NO: 8)

As an oligonucleotide having a complementary second adhesion nucleotide sequence (binding with a second oligonucleotide by complementation) comprising the nucleotide sequence capable of complementarily binding with the second adhesion nucleotide sequence for detecting the test oligonucleotide, a methylated oligonucleotide (third oligonucleotide) comprising the nucleotide sequence of SEQ ID NO: 9 to which a methylcytosine antibody is able to bind at three sites was synthesized, and a 0.1 pmoL/5 $\mu$L TE buffer solution was prepared.

<Third oligonucleotide> N represents methylated cytosine.

5'- ACGGCGCACCACGAGACTATANACANACANACAGACACAGACTGGCAAGTTGGA -3' (SEQ ID NO: 9)

Using the obtained solutions of the test oligonucleotide, the specific oligonucleotide, the first oligonucleotide, the second oligonucleotide, and the third oligonucleotide, the following three treatments (Treatment methods 1, 2 and 3) were conducted.

Treatment method 1: In a PCR tube, 10 $\mu$L of the test oligonucleotide solution prepared in the above, 5 $\mu$L of said specific oligonucleotide solution, 5 $\mu$L of said first oligonucleotide solution, 10 $\mu$L of a buffer (330 mM Tris-Acetate pH 7.9, 660 mM KOAc, 100 mM MgOAc$_2$, 5 mM Dithiothreitol), 10 $\mu$L of 1 mg/mL BSA solution and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the first oligonucleotide (that is, for forming a complex of the test oligonucleotide, the specific oligonucleotide and the first oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

Treatment method 2: In a PCR tube, 10 $\mu$L of the test oligonucleotide solution prepared in the above, 5 $\mu$L of said specific oligonucleotide solution, each 5 $\mu$L of said first oligonucleotide solution, said second oligonucleotide solution, 10 $\mu$L of a buffer (330 mM Tris-Acetate pH 7.9, 660 mM KOAc, 100 mM MgOAc$_2$, 5 mM Dithiothreitol), 10 $\mu$L of 1 mg/mL BSA solution and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the first oligonucleotide, and formation of a double strand of the first oligonucleotide and the second oligonucleotide (that is, for forming a complex of the test oligonucleotide, the specific oligonucleotide, the first oligonucleotide and the second oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

Treatment method 3: In a PCR tube, 10 μL of the test oligonucleotide solution prepared in the above, 5 μL of said specific oligonucleotide solution, 5 μL of said first oligonucleotide solution, each 5 μL of said second oligonucleotide solution and said third oligonucleotide solution, 10 μL of a buffer (330 mM Tris-Acetate pH 7.9, 660 mM KOAc, 100 mM MgOAc$_2$, 5 mM Dithiothreitol), 10 μL of 1 mg/mL BSA solution and 20 μL of 100 mM MgCl$_2$ solution were added, and further the resultant mixture was added with sterilized ultrapure water to make the liquid amount 100 μL, and mixed. Then, for causing formation of a double strand of the test oligonucleotide and the specific oligonucleotide, and concurrently causing formation of a double strand of the test oligonucleotide and the first oligonucleotide, formation of a double strand of the first oligonucleotide and the second oligonucleotide, and formation of a double strand of the second oligonucleotide and the third oligonucleotide (that is, for forming a complex of the test oligonucleotide, the specific oligonucleotide, the first oligonucleotide, the second oligonucleotide and the third second oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

The entire mixture obtained in Treatment methods 1, 2 and 3 was transferred to a 8-well strip coated with streptavidin, and left still for about 30 minutes at room temperature, to immobilize the complex of the test oligonucleotide, the specific oligonucleotide and the first oligonucleotide, and, or the second oligonucleotide, and, or the third oligonucleotide to the 8-well strip. Thereafter, the solution was removed by decantation, and each well was washed three times with 200 μL of a washing buffer [0.05% Tween20-containing phosphate buffer (1 mM KH$_2$PO$_4$ , 3 mM Na$_2$HPO$_4$ 7H$_2$O, 154 mM NaCl pH7.4)].

Each well was added with 100 μL of a primary antibody solution [methylcytosine antibody: available from AVIVA, 1 μg/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. Thereafter, each well was washed three times with 200 μL of a washing buffer [0.05% Tween20-containing phosphate buffer (1 mM KH$_2$PO$_4$ , 3 mM Na$_2$HPO$_4$ 7H$_2$O, 154 mM NaCl pH 7.4)].

Then each well was added with a secondary antibody [mouse IgG antibody Eu-N1: 0.25 μg/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. Then each well was washed three times with 200 μL of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Each well was added and mixed with 200 μL of Enhancement Solution, and shaken for 5 minutes at room temperature using a plate shaker. Thereafter, fluorescence was measured at excitation 340 nm/fluorescence 612 nm.

For analysis of data, the following method was used. For making the values of negative control solutions constant among every group, every measured value is divided by a measured value of a negative control solution of each group, and multiplied by a minimum value of negative control solution of all groups. Then a minimum value of negative control solution of all groups is subtracted from each resultant value, and the result is used as a corrected value.

$$[Corrected\ value] = [Measured\ value] \times [Minimum\ value]/[Value$$
$$of\ negative\ control\ solution\ of\ each\ group] - [Minimum\ value]$$

The result is shown in Fig. 3. Difference in detection sensitivity was not observed between the case where only the first oligonucleotide was used (Treatment method 1) and the case where the first oligonucleotide and the second oligonucleotide were used (Treatment method 2) for detecting the test oligonucleotide. It was supposed that detection sensitivity was equivalent because an unmethylated oligonucleotide is used as the second oligonucleotide, namely the number of sites to which the methylcytosine antibody is bindable (methylated cytosine) is identical. It was revealed that the detection sensitivity improved when the first oligonucleotide, the second oligonucleotide, and the third oligonucleotide are used (Treatment method 3) for detecting the test oligonucleotide. This was attributable to the fact that there are many sites to which a methylcytosine antibody is bindable (methylated cytosine) because the first oligonucleotide, the second oligonucleotide and the third oligonucleotide are linked to the test oligonucleotide.

Example 4

An oligonucleotide comprising the nucleotide sequence of SEQ ID NO: 1 was synthesized as a test oligonucleotide, and the following TE buffer solutions were prepared.

Solution A: test oligonucleotide 0 pmol/10 μL TE buffer solution (negative control solution)
Solution B: test oligonucleotide 0.003 pmoL/10 μL TE buffer solution
Solution C: test oligonucleotide 0.01 pmoL/10 μL TE buffer solution
Solution D: test oligonucleotide 0.03 pmoL/10 μL TE buffer solution

<Test oligonucleotide>

5'-AGTGACACCATCGAGAATGTCAGATCCGGATCAGAGCGCCATCTAGATGGACATGTCACTGT CTGACTACAACATCCAGA -3' (SEQ ID NO: 1)

As a specific oligonucleotide used for obtaining a test oligonucleotide, a 5'-end biotinylated oligonucleotide comprising the nucleotide sequence of SEQ ID NO: 2 was synthesized, and a 0.1 pmoL/5 μL TE buffer solution was prepared.

<5'-end biotinylated oligonucleotide>

5'- Biotin- TCTGGATGTTGTAGTCAGACAG -3' (SEQ ID NO: 2)

As an unmethylated oligonucleotide that binds with a test oligonucleotide by complementation (first oligonucleotide) for detecting the test oligonucleotide, an oligonucleotide (first oligonucleotide comprising the nucleotide sequence of SEQ ID NO: 10) was synthesized, and a 0.1 pmoL/5 μL TE buffer solution was prepared. This first oligonucleotide has a (1,1)th adhesion nucleotide sequence and a (1,2)th adhesion nucleotide sequence which are adhesion nucleotide sequences.

<First oligonucleotide>

5'-TGACATTCTCGATGGTGTCACTCACACACACACACTCGCTTCGCGGGCAGTCAACACACACA CACAGACAACGCCTCGTTCTCGG -3' (SEQ ID NO: 10)

As an oligonucleotide having a complementary (1,1)th adhesion nucleotide sequence comprising a nucleotide sequence capable of complementarily binding with the (1,1)th adhesion nucleotide sequence (binding with the first oligonucleotide by complementation) (a (2,1)th oligonucleotide) for detecting a test oligonucleotide, a methylated oligonucleotide (a (2,1)th oligonucleotide) comprising the nucleotide sequence of SEQ ID NO: 11 to which a methylcytosine antibody is bindable at 12 sites was synthesized, and a 0.1 pmoL/5 μL TE buffer solution was prepared.

<(2,1)th oligonucleotide> N represents methylated cytosine.

5' - AGCCGACGAAGGGCTTATTAGNANANANANANANANANANANANANACCGAGAACGAGGCGTTG TCT -3' (SEQ ID NO: 11)

As an oligonucleotide having a complementary (1,2)th adhesion nucleotide sequence comprising a nucleotide sequence capable of complementarily binding with the (1,2)th adhesion nucleotide sequence (binding with the first oligonucleotide by complementation) (a (2,2)th oligonucleotide) for detecting a test oligonucleotide, a methylated oligonucleotide (a (2,2)th oligonucleotide) comprising the nucleotide sequence of SEQ ID NO: 16 as the methylated oligonucleotide to which a methylcytosine antibody is bindable at 12 sites was synthesized, and a 0.1 pmoL/5 μL TE buffer solution was prepared.

<(2,2)th oligonucleotide> N represents methylated cytosine.

5'-GTTGGCCACTGCGGAGTCGCGOANANANANANANANANANANANANANATTGACTGCCCGCGAAGC GAG -3' (SEQ ID NO: 16)

Using the solutions of the test oligonucleotide, the specific oligonucleotide, the first oligonucleotide, the (2,1) th oligonucleotide, and the (2,2)th oligonucleotide, the following three treatments (Treatment method 1, 2 and 3) were conducted.

Treatment method 1: In a PCR, 10 μL of the test oligonucleotide solution prepared in the above, 5 μL of said specific oligonucleotide solution, 5 μL of said first oligonucleotide solution, 5 μL of said (2,1)th oligonucleotide solution, 10 μL of a buffer (330 mM Tris-Acetate pH 7.9, 660 mM KOAc, 100 mM MgOAc$_2$, 5 mM Dithiothreitol), 10 μL of 1 mg/mL BSA solution and 20 μL of 100 mM MgCl$_2$ solution were added, and further the resultant mixture was added with sterilized ultrapure water to make the liquid amount 100 μL, and mixed. Then, for causing formation of a double strand of the test oligonucleotide and the specific oligonucleotide, and concurrently causing formation of a double strand of the test oligonucleotide and the first oligonucleotide, and further concurrently causing formation of a double strand of the first oligonucleotide and the (2,1)th oligonucleotide (that is, for forming a complex of the test oligonucleotide, the specific oligonucleotide, the first oligonucleotide, and the (2,1)th oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

Treatment method 2: In a PCR tube, 10 µL of the test oligonucleotide solution prepared in the above, 5 µL of said specific oligonucleotide solution, 5 µL of said first oligonucleotide solution, 5 µL of said (2,2)th oligonucleotide solution, 10 µL of a buffer (330 mM Tris-Acetate pH 7.9, 660 mM KOAc, 100 mM MgOAc$_2$, 5 mM Dithiothreitol), 10 µL of 1 mg/mL BSA solution and 20 µL of 100 mM MgCl$_2$ solution were added, and further the resultant mixture was added with sterilized ultrapure water to make the liquid amount 100 µL, and mixed. Then, for causing formation of a double strand of the test oligonucleotide and the specific oligonucleotide, and concurrently causing formation of a double strand of the test oligonucleotide and the first oligonucleotide, and formation of a double strand of the first oligonucleotide and the (2,2)th oligonucleotide (that is, for forming a complex of the test oligonucleotide, the specific oligonucleotide, the first oligonucleotide, and the (2,2)th oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

Treatment method 3: In a PCR, 10 µL of the test oligonucleotide solution prepared in the above, 5 µL of said specific oligonucleotide solution, 5 µL of said first oligonucleotide solution, 5 µL of said (2,1)th oligonucleotide solution, 5 µL of said (2,2)th oligonucleotide solution, 10 µL of a buffer (330 mM Tris-Acetate pH 7.9, 660 mM KOAc, 100 mM MgOAc$_2$, 5 mM Dithiothreitol), 10 µL of 1 mg/mL BSA solution and 20 µL of 100 mM MgCl$_2$ solution were added, and further the resultant mixture was added with sterilized ultrapure water to make the liquid amount 100 µL, and mixed. Then, for causing formation of a double strand of the test oligonucleotide and the specific oligonucleotide, and concurrently causing formation of a double strand of the test oligonucleotide and the first oligonucleotide, and formation of a double strand of the first oligonucleotide and the (2,1)th oligonucleotide, and the (2,2)th oligonucleotide (that is, for forming a complex of the test oligonucleotide, the specific oligonucleotide, the first oligonucleotide, the (2,1)th oligonucleotide and the (2,2)th oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

The entire obtained mixture was transferred to a 8-well strip coated with streptavidin, and left still for about 30 minutes at room temperature, to immobilize the complex of the test oligonucleotide, the specific oligonucleotide, the first oligonucleotide and the (2,1)th oligonucleotide, and, or the (2,2)th oligonucleotide to the 8-well strip. Thereafter, the solution was removed by decantation, and each well was washed three times with 200 µL of a washing buffer [0.05% Tween20-containing phosphate buffer (1 mM KH$_2$PO$_4$, 3 mM Na$_2$HPO$_4$ 7H$_2$O, 154 mM NaCl pH7.4)].

Each well was added with 100 µL of a primary antibody [methylcytosine antibody: available from AVIVA, 1 µg/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. Thereafter, each well was washed three times with 200 µL of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Then each well was added with 100 µL of a secondary antibody [mouse IgG antibody Eu-N1: 0.25 µg/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. After leaving still, each well was washed three times with 200 µL of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Each well was added and mixed with 200 µL of Enhancement Solution, and shaken for 5 minutes at room temperature using a plate shaker. Thereafter, fluorescence was measured at excitation 340 nm/fluorescence 612 nm.

For analysis of data, the following method was used. For making the values of negative control solutions constant among every group, every measured value is divided by a measured value of a negative control solution of each group, and multiplied by a minimum value of negative control solution of all groups. Then a minimum value of negative control solution of all groups is subtracted from each resultant value, and the result is used as a corrected value.

$$[\text{Corrected value}] = [\text{Measured value}] \times [\text{Minimum value}]/[\text{Value of negative control solution of each group}] - [\text{Minimum value}]$$

The result is shown in Fig. 4. Difference in detection sensitivity was not observed between the case where the first oligonucleotide and the (2,1)th oligonucleotide were used (Treatment method 1), and the case where the first oligonucleotide and the (2,2)the oligonucleotide were used (Treatment method 2) for detecting the test oligonucleotide. This was attributable to the fact that the number of sites to which a methylcytosine antibody is bindable (methylated cytosine) was identical. It was revealed that the detection sensitivity improved when the first oligonucleotide, the (2,1)th oligonucleotide, and the (2,2)th oligonucleotide were used (Treatment method 3) for detecting the test oligonucleotide. This was attributable to the fact that there are many sites to which a methylcytosine antibody is bindable (methylated cytosine) because the complex of the first oligonucleotide, the (2,1)th oligonucleotide, and the (2,2)th oligonucleotide is formed with the test oligonucleotide.

Example 5

An oligonucleotide comprising the nucleotide sequence of SEQ ID NO: 1 was synthesized as a test oligonucleotide, and the following TE buffer solutions were prepared.

Solution A: test oligonucleotide 0 pmol/10 $\mu$L TE buffer solution (negative control solution)
Solution B: test oligonucleotide 0.003 pmoL/10 $\mu$L TE buffer solution
Solution C: test oligonucleotide 0.01 pmoL/10 $\mu$L TE buffer solution
Solution D: test oligonucleotide 0.03 pmoL/10 $\mu$L TE buffer solution

<Test oligonucleotide>

5'-AGTGACACCATCGAGAATGTCAGATCCGGATCAGAGCGCCATCTAGATGGACATGTCACTGT CTGACTACAACATCCAGA -3' (SEQ ID NO: 1)
As a specific oligonucleotide used for obtaining a test oligonucleotide, a 5'-end biotinylated oligonucleotide comprising the nucleotide sequence of SEQ ID NO: 2 was synthesized, and a 0.1 pmoL/5 $\mu$L TE buffer solution was prepared.

<5'-end biotinylated oligonucleotide>

5'- Biotin- TCTGGATGTTGTAGTCAGACAG -3' (SEQ ID NO: 2)
As an oligonucleotide that binds with a test oligonucleotide by complementation for detecting the test oligonucleotide (first oligonucleotide), a methylated oligonucleotide comprising the nucleotide sequence of SEQ ID NO: 17 to which a methylcytosine antibody is able to bind at three sites was synthesized, and a 0.1 pmoL/5 $\mu$L TE buffer solution was prepared. This first oligonucleotide has a first adhesion nucleotide sequence which is an adhesion nucleotide sequence.

<First oligonucleotide> N represents methylated cytosine.

5'-TGACATTCTCGATGGTGTCACTCACACACACACACTCGCTTCGCGGGCAGTCAACANACANA CANAGACAACGCCTCGTTCTCGG -3' (SEQ ID NO: 17)
As an oligonucleotide having a complementary first adhesion nucleotide sequence comprising a nucleotide sequence capable of complementarily binding with the first adhesion nucleotide sequence (binding with the first oligonucleotide by complementation) for detecting the test oligonucleotide (second oligonucleotide), a methylated oligonucleotide comprising the nucleotide sequence of SEQ ID NO: 16 to which a methylcytosine antibody is bindable at 12 sites was synthesized, and a 0.1 pmoL/5 $\mu$L TE buffer solution was prepared.

<Second oligonucleotide> N represents methylated cytosine.

5' - GTTGGCCACTGCGGAGTCGCGOANANANANANANANANANANANANATTGACTGCCCGCGAAGC GAG -3' (SEQ ID NO: 16)
Using the solutions of the test oligonucleotide, the specific oligonucleotide, the first oligonucleotide, and the second oligonucleotide, the following two treatments (Treatment methods 1 and 2) were conducted.
Treatment method 1: In a PCR, 10 $\mu$L of the test oligonucleotide solution prepared in the above, 5 $\mu$L of said specific oligonucleotide solution, 5 $\mu$L of said first oligonucleotide solution, 10 $\mu$L of a buffer (330 mM Tris-Acetate pH 7.9, 660 mM KOAc, 100 mM MgOAc$_2$, 5 mM Dithiothreitol), 10 $\mu$L of 1 mg/mL BSA solution and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the first oligonucleotide (that is, for forming a complex of the test oligonucleotide, the specific oligonucleotide and the first oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.
Treatment method 2: In a PCR tube, 10 $\mu$L of the test oligonucleotide solution prepared in the above, 5 $\mu$L of said specific oligonucleotide solution, 5 $\mu$L of said first oligonucleotide solution, 5 $\mu$L of said second oligonucleotide

solution, 10 $\mu$L of a buffer (330 mM Tris-Acetate pH 7.9, 660 mM KOAc, 100 mM MgOAc$_2$, 5 mM Dithiothreitol), 10 $\mu$L of 1 mg/mL BSA solution and 20 $\mu$L of 100 mM MgCl$_2$ solution were added, and further the resultant mixture was added with sterilized ultrapure water to make the liquid amount 100 $\mu$L, and mixed. Then, for causing formation of a double strand of the test oligonucleotide and the specific oligonucleotide, and concurrently causing formation of a double strand of the test oligonucleotide and the first oligonucleotide, and formation of a double strand of the first oligonucleotide and the second oligonucleotide (that is, for forming a complex of the test oligonucleotide, the specific oligonucleotide, the first oligonucleotide and the second oligonucleotide), the PCR tube was heated at 95°C for 10 minutes, rapidly cooled to 70°C and kept at this temperature for 10 minutes. Then the reaction was cooled to 50°C and retained for 10 minutes, and further retained at 37°C for 10 minutes, and then returned to room temperature.

The entire obtained mixture was transferred to a 8-well strip coated with streptavidin, and left still for about 30 minutes at room temperature, to immobilize the complex of the test oligonucleotide, the specific oligonucleotide and the first oligonucleotide, or the test oligonucleotide, the specific oligonucleotide, the first oligonucleotide and the second oligonucleotide to the 8-well strip. Thereafter, the solution was removed by decantation, and each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH7.4)].

Each well was added with 100 $\mu$L of a primary antibody [methylcytosine antibody: available from AVIVA, 1 $\mu$g/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. Thereafter, each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Then, each well was added with a secondary antibody solution [mouse IgG antibody FITC (derived from goat): available from MBL, 2 $\mu$g/mL 0.1% BSA-containing phosphate buffer (1 mM KH$_2$PO$_4$, 3 mM Na$_2$HPO$_4$ 7H$_2$O, 154 mM NaCl pH 7.4) solution], and left still for 1 hour at room temperature. After leaving still, each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Each well was added with 100 $\mu$L of a tertiary antibody solution [Peroxidase-conjugated IgG Fraction Monoclonal Mouse Anti-Fluorescein: available from Jackson ImmunoResearch Laboratories, 0.1 $\mu$g/mL 0.1% BSA-containing phosphate buffer (1 mM KH$_2$ PO$_4$ , 3 mM Na$_2$HPO$_4$ 7H$_2$O, 154 mM NaCl pH 7.4) solution], and left still for 1 hour at room temperature. Then each well was washed three times with 200 $\mu$L of a washing buffer [0.05% Tween20-containing phosphate buffer (1 mM KH$_2$PO$_4$, 3 mM Na$_2$HPO$_4$ 7H$_2$O, 154 mM NaCl pH 7.4)].

Each well was added and mixed with 100 $\mu$L of a substrate (available from R&D systems Inc., #DY999), to initiate the reaction.

After leaving still for about 5 minutes at room temperature, each well was added with 50 $\mu$L of a stop solution (1N H$_2$SO$_4$ aqueous solution), to stop the reaction. Within 30 minutes after stopping of the reaction, absorbance at 450 nm was measured.

For analysis of data, the following method was used. For making the values of negative control solutions constant among every group, every measured value is divided by a measured value of a negative control solution of each group, and multiplied by a minimum value of negative control solution of all groups. Then a minimum value of negative control solution of all groups is subtracted from each resultant value, and the result is used as a corrected value.

```
[Corrected value] = [Measured value] x [Minimum value]/[Value

of negative control solution of each group] - [Minimum value]
```

The result is shown in Fig. 5. It was revealed that detection sensitivity improved in Treatment method 2 when only the first oligonucleotide was used (Treatment method 1) and when the first oligonucleotide and the second oligonucleotide were used (Treatment method 2) for detecting the test oligonucleotide. This was attributable to the fact that there are many sites to which a methylcytosine antibody is bindable (methylated cytosine) by forming a complex of the first oligonucleotide and the second oligonucleotide with the test oligonucleotide.

Industrial Applicability

According to the present invention, it becomes possible to provide a method for quantifying or detecting DNA comprising a target DNA region contained in a specimen in a simple and convenient manner, and so on.

Free Text in Sequence Listing

SEQ ID NO:1
Designed oligonucleotide
SEQ ID NO:2
Designed biotinated oligonucleotide for fixation
SEQ ID NO:3
Designed FITC labeled oligonucleotide
SEQ ID NO:4
Designed FITC labeled oligonucleotide
SEQ ID NO:5
Designed oligonucleotide
SEQ ID NO:6
Designed oligonucleotide
SEQ ID NO:7
Designed oligonucleotide
SEQ ID NO:8
Designed oligonucleotide
SEQ ID NO:9
Designed oligonucleotide
SEQ ID NO:10
Designed oligonucleotide
SEQ ID NO:11
Designed oligonucleotide
SEQ ID NO:16
Designed oligonucleotide
SEQ ID NO:17
Designed oligonucleotide

**Claims**

1. A method for quantifying or detecting DNA which comprises a target DNA region and is contained in a specimen, comprising:

   (1) A first step of preparing a specimen containing a test oligonucleotide that is DNA comprising a target DNA region;
   (2) A second step of mixing the test oligonucleotide contained in the specimen prepared in the first step and a detection oligonucleotide that is capable of complementarily binding with the test oligonucleotide and has a plurality of identification functions, to form a detection complex comprising the test oligonucleotide and the detection oligonucleotide, and immobilizing the detection complex to a support; and
   (3) A third step of quantifying or detecting said DNA comprising a target DNA region by detecting the detection oligonucleotide contained in the detection complex formed in the second step by its identification function.

2. The method according to claim 1, wherein the detection oligonucleotide having a plurality of identification functions is a composite detection oligonucleotide comprising a plurality of complementarily bound oligonucleotides, or a composite detection oligonucleotide comprising a methylated oligonucleotide having a plurality of methylation sites.

3. The method according to claim 2, wherein the composite detection oligonucleotide comprises an oligonucleotide comprising methylated DNA.

4. The method according to claim 2 or 3, wherein the identification function of the composite detection oligonucleotide is identification function of a bound methylated DNA antibody.

5. The method according to claim 3 or 4, wherein the methylated DNA is 5-methylcytosine.

6. The method according to claim 4 or 5, wherein the methylated DNA antibody is methylcytosine antibody.

7. The method according to any one of claims 1 to 6, wherein the specimen is any of the following biological specimens:

(a) mammalian blood, a body fluid, excreta, a body secretion, a cell lysate or a tissue lysate,
(b) DNA extracted from one selected from the group consisting of mammalian blood, a body fluid, excreta, a body secretion, a cell lysate or a tissue lysate,
(c) DNA prepared using RNA extracted from one selected from the group consisting of mammalian tissue, cell, tissue lysate or cell lysate as a template,
(d) DNA extracted from a bacterium, a fungus or a virus, or
(f) DNA prepared using RNA extracted from a bacterium, a fungus, or a virus as a template.

8. A method for labeling a specimen using the composite detection oligonucleotide used in the method of any one of claims 2 to 7.

9. A method for labeling a specimen using the composite detection oligonucleotide used in the method of any one of claims 2 to 7 and a reagent capable of labeling the composite detection oligonucleotide.

10. The method according to claim 8 or 9, wherein an object to be labeled is DNA or protein.

11. The method according to any one of claims 1 to 10, wherein in the second step, the detection complex and the support are bound via a specific oligonucleotide that does not inhibit binding of the test oligonucleotide and the detection oligonucleotide, and is capable of complementarily binding with the test oligonucleotide.

12. The method according to any one of claims 1 to 11, wherein the DNA comprising a target DNA region is any of the following DNA comprising a target DNA region:

(a) DNA digested in advance with a restriction enzyme recognition cleavage site for which is not present in the target DNA region,
(b) DNA comprising a target DNA region and being purified in advance,
(c) free DNA comprising a target DNA region in blood,
(d) DNA comprising a target DNA region and being derived from microbial genome, or
(e) DNA comprising a target DNA region and having been generated from RNA by a reverse transcriptase.

13. A method for quantifying or detecting DNA which comprises a target DNA region and is contained in a specimen, comprising:

(1) A first step of preparing a specimen containing a test oligonucleotide that is DNA comprising a target DNA region,
(2) A second step of mixing the test oligonucleotide contained in the specimen prepared in the first step, and a detection oligonucleotide that is capable of complementarily binding with the test oligonucleotide and comprises a methylated oligonucleotide, to form a detection complex comprising the test oligonucleotide and the detection oligonucleotide, and immobilizing the detection complex to a support, and
(3) A third step of quantifying or detecting said DNA comprising a target DNA region by detecting the detection oligonucleotide contained in the detection complex formed in the second step by its identification function.

14. The method according to claim 13, wherein the identification function employs a methylated DNA antibody or methylcytosine antibody.

Fig.1

Fig.2

Fig.3

Fig.4

Fig.5

## INTERNATIONAL SEARCH REPORT

| International application No. |
| --- |
| PCT/JP2009/064686 |

A. CLASSIFICATION OF SUBJECT MATTER
C12Q1/68(2006.01)i, G01N33/58(2006.01)i, C12N15/09(2006.01)n, G01N33/53
(2006.01)n

According to International Patent Classification (IPC) or to both national classification and IPC

B. FIELDS SEARCHED

Minimum documentation searched (classification system followed by classification symbols)
C12Q1/68, G01N33/58, C12N15/09, G01N33/53

Documentation searched other than minimum documentation to the extent that such documents are included in the fields searched

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X/A | WO 87/03622 A1 (PRINCETON UNIVERSITY), 18 June, 1987 (18.06.87), Full text & JP 63-501984 A & US 5424188 A & EP 248896 A | 1,2,7-12/ 3-6,13,14 |
| A | JP 2000-515367 A (Tm Technologies, Inc.), 21 November, 2000 (21.11.00), Full text; particularly, Fig. 1 & US 5902724 A & EP 938588 A & WO 98/002580 A2 | 1-14 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 02 September, 2009 (02.09.09) | 15 September, 2009 (15.09.09) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**EP 2 327 799 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *J. Cataract. Refract. Surg.,* 2007, vol. 33 (4), 635-641
- *Environ. Mol. Mutagen.,* 1991, vol. 18 (4), 259-262
- Methods in Yeast Genetics. Cold Spring Harbor Laboratory Press
- Molecular Cloning - A Laboratory Manual. Cold Spring Harbor Laboratory Press
- *J. Am. Chem. Soc.,* 2007, vol. 129, 5612-5620
- Molecular Cloning, A Laboratory Manual. Cold Spring Harbor Laboratory, 1989


SEQUENCE LISTING

<110> Sumitomo Chemical Co., Ltd.

<120> Method for determining or detecting DNA

<130> S21224W001

<150> JP2008-210467
<151> 2008-08-19

<160> 17

<210> 1
<211> 80
<212> DNA
<213> Artificial Sequence

<220>
<223> Designed oligonucleotide

<400> 1
agtgacacca tcgagaatgt cagatccgga tcagagcgcc atctagatgg acatgtcact    60
gtctgactac aacatccaga                                                 80

<210> 2
<211> 22
<212> DNA
<213> Artificial Sequence

<220>
<223> Designed biotinated oligonucleotide for fixation

<400> 2

tctggatgtt gtagtcagac ag                                                    22

<210> 3

<211> 22

<212> DNA

<213> Artificial Sequence


<220>

<223> Designed FITC labeled oligonucleotide


<400> 3

tgacattctc gatggtgtca ct                                                    22


<210> 4

<211> 65

<212> DNA

<213> Artificial Sequence


<220>

<223> Designed FLC labeled oligonucleotide


<400> 4

tgacattctc gatggtgtca ctcacacaca cacacacaca cacacagaca acgcctcgtt          60

ctcgg                                                                       65


<210> 5

<211> 65

<212> DNA

<213> Artificial Sequence


<220>

<223> Designed oligonucleotide

<210> 5

<220>

<221> modified_base

<222> 45

<223> n=m5c


<400> 5

tgacattctc gatggtgtca ctcacacaca cacacacaca cacanagaca acgcctcgtt 60

ctcgg 65


<210> 6

<211> 65

<212> DNA

<213> Artificial Sequence


<220>

<223> Designed oligonucleotide

<220>

<221> modified_base

<222> 23, 25, 27, 29, 31, 33, 35, 37, 39, 41, 43, 45

<223> n=m5c


<400> 6

tgacattctc gatggtgtca ctnananana nanananana nananagaca acgcctcgtt 60

ctcgg 65


<210> 7

<211> 51

<212> DNA

<213> Artificial Sequence


<220>

<223> Designed oligonucleotide

<220>

<210> modified_base

<222> 24, 28, 32

<223> n=m5c


<400> 7

tgacattctc gatggtgtca ctanacanac anatgcgcac cgtgcgcgag c          51


<210> 8

<211> 49

<212> DNA

<213> Artificial Sequence


<220>

<223> Designed oligonucleotide


<400> 8

atagtctcgt ggtgcgccgt acacacacac agctcgcgca cggtgcgca          49


<210> 9

<211> 54

<212> DNA

<213> Artificial Sequence


<220>

<223> Designed oligonucleotide

<220>

<221> modified_base

<222> 22, 26, 30

<223> n=m5c


<400> 9

acggcgcacc acgagactat anacanacan acagacacag actggcaagt tgga          54

<210> 10

<211> 85

<212> DNA

<213> Artificial Sequence


<220>

<223> Designed oligonucleotide


<400> 10

tgacattctc gatggtgtca ctcacacaca cacactcgct tcgcgggcag tcaacacaca     60

cacacagaca acgcctcgtt ctcgg     85


<210> 11

<211> 65

<212> DNA

<213> Artificial Sequence


<220>

<223> Designed oligonucleotide

<220>

<221> modified_base

<222> 22, 24, 26, 28, 30, 32, 34, 36, 38, 40, 42, 44

<223> n=m5c


<400> 11

agccgacgaa gggcttatta gnanananan ananananan ananaccgag aacgaggcgt     60

tgtct     65


<210> 12

<211> 271

<212> DNA

<213> Homo sapiens

<220>

<223> Genbank Accession No. M80340 115-386

<400> 12

```
tagggagtgc cagacagtgg gcgcaggcca gtgtgtgtgc gcaccgtgcg cgagccgaag    60
cagggcgagg cattgcctca cctgggaagc gcaaggggtc agggagttcc ctttctgagt   120
caaagaaagg ggtgacggtc gcacctggaa aatcgggtca ctcccacccg aatattgcgc   180
ttttcagacc ggcttaagaa acggcgcacc acgagactat atcccacacc tggctcggag   240
ggtcctacgc ccacggaatc tcgctgattg c                                  271
```

<210> 13

<211> 332

<212> DNA

<213> Homo sapiens

<400> 13

```
tagaatatcc aatacagaga agtgcttaaa ggagctgatg gagctgaaaa ccaaggctcg    60
agaactacgt gaagaatgca gaagcctcag gagccgatgc gatcaactgg aagaaagggt   120
atcagcaatg gaagatgaaa tgaatgaaat gaagcgagaa gggaagttta gagaaaaaag   180
aataaaaaga aatgagcaaa gcctccaaga aatatgggac tatgtgaaaa gaccaaatct   240
acgtctgatt ggtgtacctg aaagtgatgt ggagaatgga accaagttgg aaaacactct   300
gcaggatatt atccaggaga acttccccaa tc                                 332
```

<210> 14

<211> 267

<212> DNA

<213> Homo sapiens

<400> 14

```
tagaactcag gattaagaat ctcactcaaa gccgctcaac tacatggaaa ctgaacaacc    60
tgctcctgaa tgactactgg gtacataacg aaatgaaggc agaaataaag atgttctttg   120
aaaccaacga gaacaaagac accacatacc agaatctctg ggacgcattc aaagcagtgt   180
```

gtagagggaa atttatagca ctaaatgcct acaagagaaa gcaggaaaga tccaaaattg    240

acaccctaac atcacaatta aaagaac    267


<210> 15

<211> 85

<212> DNA

<213> Homo sapiens


<220>

<223> Genbank Accession No. AF458110 178-262


<400> 15

cgggcgcggt ggctcacgcc tgtaatccca gcactttggg aggccgaggt gggcggatca    60

cgaggtcagg agatcgagac catcc    85


<210> 16

<211> 65

<212> DNA

<213> Artificial Sequence


<220>

<223> Designed oligonucleotide

<220>

<221> modified_base

<222> 24, 26, 28, 30, 32, 34, 36, 38, 40, 42, 44

<223> n=m5c


<400> 16

gttggccact gcggagtcgc goanananan anananananan ananattgac tgcccgcgaa    60

gcgag    65


<210> 17

<211> 85

<212> DNA

<213> Artificial Sequence

<220>

<223> Designed oligonucleotide

<220>

<221> modified_base

<222> 57, 61, 65

<223> n=m5c

<400> 17

tgacattctc gatggtgtca ctcacacaca cacactcgct tcgcgggcag tcaacanaca    60

nacanagaca acgcctcgtt ctcgg    85

SEQUENCE LISTING

<110> Sumitomo Chemical Co., Ltd.

<120> Method for determining or detecting DNA

<130> S21224WO01

<150> JP2008-210467
<151> 2008-08-19

<160> 17

<210> 1
<211> 80
<212> DNA
<213> Artificial Sequence

<220>
<223> Designed oligonucleotide

<400> 1
agtgacacca tcgagaatgt cagatccgga tcagagcgcc atctagatgg acatgtcact    60
gtctgactac aacatccaga                                                 80

<210> 2
<211> 22
<212> DNA
<213> Artificial Sequence

<220>
<223> Designed biotinated oligonucleotide for fixation

<400> 2

tctggatgtt gtagtcagac ag                                                    22


<210> 3

<211> 22

<212> DNA

<213> Artificial Sequence


<220>

<223> Designed FITC labeled oligonucleotide


<400> 3

tgacattctc gatggtgtca ct                                                    22


<210> 4

<211> 65

<212> DNA

<213> Artificial Sequence


<220>

<223> Designed FLC labeled oligonucleotide


<400> 4

tgacattctc gatggtgtca ctcacacaca cacacacaca cacacagaca acgcctcgtt    60

ctcgg                                                                       65


<210> 5

<211> 65

<212> DNA

<213> Artificial Sequence


<220>

<223> Designed oligonucleotide

<220>

<221> modified_base

<222> 45

<223> n=m5c


<400> 5

tgacattctc gatggtgtca ctcacacaca cacacacaca cacanagaca acgcctcgtt  60

ctcgg  65


<210> 6

<211> 65

<212> DNA

<213> Artificial Sequence


<220>

<223> Designed oligonucleotide

<220>

<221> modified_base

<222> 23, 25, 27, 29, 31, 33, 35, 37, 39, 41, 43, 45

<223> n=m5c


<400> 6

tgacattctc gatggtgtca ctnananana nanananana nananagaca acgcctcgtt  60

ctcgg  65


<210> 7

<211> 51

<212> DNA

<213> Artificial Sequence


<220>

<223> Designed oligonucleotide

<220>

<210> modified_base

<222> 24, 28, 32

<223> n=m5c


<400> 7

tgacattctc gatggtgtca ctanacanac anatgcgcac cgtgcgcgag c          51


<210> 8

<211> 49

<212> DNA

<213> Artificial Sequence


<220>

<223> Designed oligonucleotide


<400> 8

atagtctcgt ggtgcgccgt acacacacac agctcgcgca cggtgcgca          49


<210> 9

<211> 54

<212> DNA

<213> Artificial Sequence


<220>

<223> Designed oligonucleotide

<220>

<221> modified_base

<222> 22, 26, 30

<223> n=m5c


<400> 9

acggcgcacc acgagactat anacanacan acagacacag actggcaagt tgga          54

<210> 10

<211> 85

<212> DNA

<213> Artificial Sequence


<220>

<223> Designed oligonucleotide


<400> 10

tgacattctc gatggtgtca ctcacacaca cacactcgct tcgcgggcag tcaacacaca    60

cacacagaca acgcctcgtt ctcgg    85


<210> 11

<211> 65

<212> DNA

<213> Artificial Sequence


<220>

<223> Designed oligonucleotide

<220>

<221> modified_base

<222> 22, 24, 26, 28, 30, 32, 34, 36, 38, 40, 42, 44

<223> n=m5c


<400> 11

agccgacgaa gggcttatta gnanananan ananananan ananaccgag aacgaggcgt    60

tgtct    65


<210> 12

<211> 271

<212> DNA

<213> Homo sapiens

<220>

<223> Genbank Accession No. M80340 115-386

<400> 12

```
tagggagtgc cagacagtgg gcgcaggcca gtgtgtgtgc gcaccgtgcg cgagccgaag      60
cagggcgagg cattgcctca cctgggaagc gcaaggggtc agggagttcc ctttctgagt     120
caaagaaagg ggtgacggtc gcacctggaa aatcgggtca ctcccacccg aatattgcgc     180
ttttcagacc ggcttaagaa acggcgcacc acgagactat atcccacacc tggctcggag     240
ggtcctacgc ccacggaatc tcgctgattg c                                    271
```

<210> 13

<211> 332

<212> DNA

<213> Homo sapiens

<400> 13

```
tagaatatcc aatacagaga agtgcttaaa ggagctgatg gagctgaaaa ccaaggctcg      60
agaactacgt gaagaatgca gaagcctcag gagccgatgc gatcaactgg aagaaagggt     120
atcagcaatg gaagatgaaa tgaatgaaat gaagcgagaa gggaagttta gagaaaaaag     180
aataaaaaga aatgagcaaa gcctccaaga aatatgggac tatgtgaaaa gaccaaatct     240
acgtctgatt ggtgtacctg aaagtgatgt ggagaatgga accaagttgg aaaacactct     300
gcaggatatt atccaggaga acttccccaa tc                                   332
```

<210> 14

<211> 267

<212> DNA

<213> Homo sapiens

<400> 14

```
tagaactcag gattaagaat ctcactcaaa gccgctcaac tacatggaaa ctgaacaacc      60
tgctcctgaa tgactactgg gtacataacg aaatgaaggc agaaataaag atgttctttg     120
aaaccaacga gaacaaagac accacatacc agaatctctg ggacgcattc aaagcagtgt     180
```

gtagagggaa atttatagca ctaaatgcct acaagagaaa gcaggaaaga tccaaaattg    240

acaccctaac atcacaatta aaagaac    267


<210> 15

<211> 85

<212> DNA

<213> Homo sapiens


<220>

<223> Genbank Accession No. AF458110 178-262


<400> 15

cgggcgcggt ggctcacgcc tgtaatccca gcactttggg aggccgaggt gggcggatca    60

cgaggtcagg agatcgagac catcc    85


<210> 16

<211> 65

<212> DNA

<213> Artificial Sequence


<220>

<223> Designed oligonucleotide

<220>

<221> modified_base

<222> 24, 26, 28, 30, 32, 34, 36, 38, 40, 42, 44

<223> n=m5c


<400> 16

gttggccact gcggagtcgc goanananan ananananan ananattgac tgcccgcgaa    60

gcgag    65


<210> 17

<211> 85

<212> DNA

<213> Artificial Sequence


<220>

<223> Designed oligonucleotide

<220>

<221> modified_base

<222> 57, 61, 65

<223> n=m5c


<400> 17

tgacattctc gatggtgtca ctcacacaca cacactcgct tcgcgggcag tcaacanaca    60

nacanagaca acgcctcgtt ctcgg    85